(19) **Europäisches Patentamt**
**European Patent Office**
**Office européen des brevets**

(11) **EP 4 122 488 A1**

(12) **EUROPEAN PATENT APPLICATION**
published in accordance with Art. 153(4) EPC

(43) Date of publication:
**25.01.2023 Bulletin 2023/04**

(21) Application number: **21774054.7**

(22) Date of filing: **24.03.2021**

(51) International Patent Classification (IPC):
**A61K 38/43** (2006.01)    **A61K 38/49** (2006.01)
**A61P 25/28** (2006.01)

(52) Cooperative Patent Classification (CPC):
**A61K 38/43; A61K 38/49;** A61P 25/28

(86) International application number:
**PCT/CN2021/082701**

(87) International publication number:
**WO 2021/190558 (30.09.2021 Gazette 2021/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.03.2020 CN 202010213462**

(71) Applicant: **Talengen International Limited**
**Hong Kong 999077 (HK)**

(72) Inventor: **LI, Jinan**
**Beijing 100089 (CN)**

(74) Representative: **karo IP**
**karo IP Patentanwälte**
**Kahlhöfer Rößler Kreuels PartG mbB**
**Postfach 32 01 02**
**40416 Düsseldorf (DE)**

Remarks:
The complete document including Reference Table(s) and the Sequence Listing(s) can be downloaded from the EPO website

(54) **METHOD AND DRUG FOR TREATING ALZHEIMER DISEASE**

(57)    The present invention provides a method and drug for preventing and treating Alzheimer's disease. The method includes: administering a therapeutically effective amount of a component of a plasminogen activation pathway to a subject. The present invention also provides a drug, pharmaceutical composition, product, and kit containing the component of the plasminogen activation pathway.

1      **EP 4 122 488 A1**      2

## Description

FIELD OF THE INVENTION

The present invention relates to a method for preventing or treating Alzheimer's disease, which includes: administering a therapeutically effective amount of a component of a plasminogen activation pathway or its related compound, such as plasminogen, to a subject to improve clinical symptoms and physical signs.

BACKGROUND OF THE INVENTION

Alzheimer's disease (AD) is a progressive neurodegenerative disease with insidious onset. It is characterized clinically by generalized dementia symptoms such as memory impairment, aphasia, apraxia, agnosia, visuospatial skill damage, executive dysfunction, and personality and behavior changes, and its cause is still unknown. The main symptoms are cognitive decline, mental symptoms and behavior disturbances, and progressive decline in daily living abilities. The course of Alzheimer's disease is divided into three stages according to the degree of deterioration of cognitive ability and physical function. The first stage, usually 1 to 3 years, is called a mild dementia stage. Patients at this stage show memory loss, prominent forgetfulness of recent events, and decreased judgement ability. They are unable to analyze, think, and judge events, and have difficulty in dealing with complex problems. They are inattentive to work or household chores, are unable to carry out shopping, financial affairs, and the like independently, and have difficulty in social activities. Although they can still do some familiar daily tasks, they show bewilderment and difficulty in understanding new things, emotional indifference, occasional irritation, and frequent paranoia. They present with time disorientation, can orient to places and people, have difficulty in orientation to geographical locations, and have poor visuospatial ability for complex structures. They have low verbal vocabulary and difficulty in naming. The second stage, usually 2 to 10 years, is called a moderate dementia stage. Patients at this stage show severe remote and recent memory impairment, declined visuospatial ability for simple structures, and time and place disorientation. They have severe impairment in handling problems and identifying similarities and differences of things. They are unable to perform outdoor activities independently, and need assistance in dressing, personal hygiene, and maintaining personal appearance. They are unable to perform calculations. They develop various neurologic symptoms, such as aphasia, apraxia, and agnosia. Their emotions change from indifference to irritability, and they often walk incessantly and may have uroclepsia. The third stage, usually 8 to 12 years, is called a severe dementia stage. Patients are completely dependent on caregivers, and have severe memory loss with only fragmented memories. They are unable to take care of themselves in daily life, have incontinence, mutism, and limb rigidity, show positive pyramidal signs on physical examination results, and show primitive reflexes such as grasping, groping, and sucking. The patients eventually fall into a coma and usually die from complications such as infection.

The current treatment method is mainly symptomatic and controls psychopathological symptoms associated with Alzheimer's disease. For example, antianxiety drugs are administered for anxiety, agitation, and insomnia; antidepressants are administered for depression; and antipsychotic drugs are administered to control behavior disorders in patients. In addition, in order to improve cognitive function and delay disease progression, nootropic drugs or drugs for improving cognitive function, such as drugs acting on neurotransmitters, cerebral vasodilators, and drugs for promoting cerebral metabolism, are administered. It is necessary to develop other treatment methods and drugs for treating Alzheimer's disease.

SUMMARY OF THE INVENTION

The present invention finds that plasminogen can promote the recovery of memory function in patients with Alzheimer's disease, improve cognitive ability, significantly reduce and relieve various clinical symptoms and physical signs of patients with Alzheimer's disease, and prevent and treat Alzheimer's disease.

Specifically, the present invention relates to the following items.

    1. In an aspect, the present invention relates to a method for preventing and treating Alzheimer's disease, which includes: administering a therapeutically effective amount of one or more compounds to a subject with Alzheimer's disease. The one or more compounds are selected from: a component of a plasminogen activation pathway, a compound capable of directly activating plasminogen or indirectly activating plasminogen by activating an upstream component of a plasminogen activation pathway, a compound mimicking the activity of plasminogen or plasmin, a compound capable of up-regulating the expression of plasminogen or a plasminogen activator, a plasminogen analog, a plasmin analog, a tPA or uPA analog, and an antagonist of a fibrinolysis inhibitor.

In an aspect, the present invention relates to use of one or more compounds in preparation of a drug for treating Alzheimer's disease. The one or more compounds are selected from: a component of a plasminogen activation pathway, a compound capable of directly activating plasminogen or indirectly activating plasminogen by activating an upstream component of a plasminogen activation pathway, a compound mimicking the activity of plasminogen or plasmin, a compound capable of up-regulating the expression of plasminogen or a plasmino-

2

gen activator, a plasminogen analog, a plasmin analog, a tPA or uPA analog, and an antagonist of a fibrinolysis inhibitor.

In an aspect, the present invention relates to a drug or pharmaceutical composition for treating Alzheimer's disease that contains one or more compounds. The one or more compounds are selected from: a component of a plasminogen activation pathway, a compound capable of directly activating plasminogen or indirectly activating plasminogen by activating an upstream component of a plasminogen activation pathway, a compound mimicking the activity of plasminogen or plasmin, a compound capable of up-regulating the expression of plasminogen or a plasminogen activator, a plasminogen analog, a plasmin analog, a tPA or uPA analog, and an antagonist of a fibrinolysis inhibitor.

2. The method, the use, the drug or the pharmaceutical composition according to item 1, wherein the component of the plasminogen activation pathway is selected from plasminogen, recombinant human plasmin, Lys-plasminogen, Glu-plasminogen, plasmin, plasminogen and plasmin variants and analogs containing one or more kringle domains and protease domains of plasminogen and plasmin, mini-plasminogen, mini-plasmin, micro-plasminogen, micro-plasmin, delta-plasminogen, delta-plasmin, a plasminogen activator, tPA, and uPA.

3. The method, the use, the drug or the pharmaceutical composition according to item 1, wherein the antagonist of the fibrinolysis inhibitor is an inhibitor of PAI-1, a complement C1 inhibitor, $\alpha$2 antiplasmin or an $\alpha$2 macroglobulin, such as an antibody.

4. The method, the use, the drug or the pharmaceutical composition according to any one of items 1 to 3, wherein the compound has one or more effects on the subject with Alzheimer's disease. The one or more effects are selected from: promotion of the degradation of amyloid beta-protein 40 (A$\beta$40) or amyloid beta-protein 42 (A$\beta$42) in brain tissue, improvement of memory function, improvement of cognitive ability, improvement of geographical identification ability, relief of anxiety or depression, reduction of A$\beta$42 deposition in brain tissue, promotion of the degradation of Tau proteins in brain tissue, promotion of the cleavage of Pro-BDNF in brain tissue to form mature BDNF, promotion of the expression of BDNF in brain tissue, promotion of the cleavage of Pro-NGF in brain tissue to form mature NGF, and improvement of hippocampal damage in brain tissue.

5. The method, the use, the drug or the pharmaceutical composition according to any one of items 1 to 4, wherein the compound is plasminogen.

6. The method, the use, the drug or the pharmaceutical composition according to any one of items 1 to 5, wherein the plasminogen is human full-length plasminogen or a conservatively substituted variant thereof.

7. The method, the use, the drug or the pharmaceutical composition according to any one of items 1 to 5, wherein the plasminogen has at least 75%, 80%, 85%, 90%, 95%, 96%, 97%, 98% or 99% sequence identity with sequence 2 and still has the lysine binding activity or the proteolytic activity of plasminogen.

8. The method, the use, the drug or the pharmaceutical composition according to any one of items 1 to 5, wherein the plasminogen is a protein containing an amino acid sequence that has at least 80%, 90%, 95%, 96%, 97%, 98% or 99% amino acid sequence identity with sequence 14 and still having the proteolytic activity of plasminogen.

9. The method, the use, the drug or the pharmaceutical composition according to any one of items 1 to 5, wherein the plasminogen is selected from Glu-plasminogen, Lys-plasminogen, mini-plasminogen, micro-plasminogen, delta-plasminogen, and variants thereof that retain the proteolytic activity of plasminogen.

10. The method, the use, the drug or the pharmaceutical composition according to any one of items 1 to 5, wherein the plasminogen contains an amino acid sequence shown as sequence 2, 6, 8, 10 or 12, or contains a conservatively substituted variant of the amino acid sequence shown as sequence 2, 6, 8, 10 or 12.

11. The method, the use, the drug or the pharmaceutical composition according to any one of items 1 to 10, wherein the compound is used in combination with one or more other treatment methods or drugs.

12. The method, the use, the drug or the pharmaceutical composition according to item 11, wherein the other treatment methods include a cell therapy (including a stem cell therapy), a support therapy, and a physical therapy.

13. The method, the use, the drug or the pharmaceutical composition according to item 11, wherein the other drugs are other drugs for treating Alzheimer's disease.

14. The method, the use, the drug or the pharmaceutical composition according to any one of items 1 to 13, wherein the compound is administered by nasal inhalation, aerosol inhalation, nasal drops, eye drops, ear drops, an intravenous method, an intraperitoneal method, a subcutaneous method, an intracranial method, an intrathecal method, an intraarterial method (e.g. via the carotid artery) or an intramuscular method.

In any one of the above embodiments of the present invention, the plasminogen may have at least 75%, 80%, 85%, 90%, 95%, 96%, 97%, 98% or 99% sequence identity with sequence 2, 6, 8, 10 or 12, and still have the activity, such as the lysine binding activity and the proteolytic activity, of plasminogen. In some embodiments, the plasminogen is a protein that is obtained by adding, deleting and/or substituting 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid on the basis of sequence 2, 6, 8, 10 or 12, and still has the activity, such as the lysine binding activity and the proteolytic activity, of plasminogen.

In some embodiments, the plasminogen is a protein containing a plasminogen active fragment and still having the activity, such as the proteolytic activity, of plasminogen. In some embodiments, the plasminogen is selected from Glu-plasminogen, Lys-plasminogen, mini-plasminogen, micro-plasminogen, delta-plasminogen, and variants thereof that retain the activity, such as the proteolytic activity, of plasminogen. In some embodiments, the plasminogen is natural or synthesized human plasminogen, or a variant or fragment thereof that still retains the activity, such as the lysine binding activity and the proteolytic activity, of plasminogen. In some embodiments, the plasminogen is a human plasminogen ortholog from a primate or a rodent, or a variant or fragment thereof that retains the activity, such as the lysine binding activity and the proteolytic activity, of plasminogen. In some embodiments, the plasminogen has an amino acid sequence shown as sequence 2, 6, 8, 10 or 12. In some embodiments, the plasminogen is human natural plasminogen.

In some embodiments, the subject is a human. In some embodiments, the subject has the plasminogen deficiency. In some embodiments, the deficiency is congenital, secondary and/or local.

In some embodiments, the pharmaceutical composition contains a pharmaceutically acceptable carrier and plasminogen used in the above method. In some embodiments, the kit may be a preventative or therapeutic kit, which includes: (i) plasminogen used in the above method and (ii) a means for delivering the plasminogen to the subject. In some implementation, the means is a syringe or a vial. In some embodiments, the kit also contains a label or instructions. The label or the instructions indicate that the plasminogen is administered to the subject to implement any one of the above methods.

In some embodiments, the product contains: a container with a label, and (i) plasminogen used in the above method or a pharmaceutical composition containing plasminogen. The label indicates that the plasminogen or the composition is administered to the subject to implement any one of the above methods.

In some embodiments, the kit or the product also contains other one or more means or containers. The means or the containers contain other drugs.

In some embodiments of the above method, the plasminogen is administered systemically or locally, and preferably, the plasminogen is administered intravenously, intramuscularly or subcutaneously to treat the subject. In some embodiments of the above method, the plasminogen is administered in combination with a suitable polypeptide carrier or stabilizer. In some embodiments of the above method, the plasminogen is daily administered at a dose of 0.0001-2000 mg/kg, 0.001-800 mg/kg, 0.01-600 mg/kg, 0.1-400 mg/kg, 1-200 mg/kg, 1-100 mg/kg or 10-100 mg/kg (based on per kilogram of body weight), or is daily administered at a dose of 0.0001-2000 mg/cm$^2$, 0.001-800 mg/cm$^2$, 0.01-600 mg/cm$^2$, 0.1-400 mg/cm$^2$, 1-200 mg/cm$^2$, 1-100 mg/cm$^2$ or 10-100 mg/cm$^2$ (based on per square centimetre of body surface area), preferably, administration is repeated at least once, and preferably, administration is performed at least daily.

The present invention explicitly covers all combinations of the technical features belonging to the embodiments of the present invention, and the combined technical solutions have been explicitly disclosed in the present invention, just as the above technical solutions have been separately and explicitly disclosed. In addition, the present invention also explicitly covers combinations of the embodiments and their elements, and the combined technical solutions are explicitly disclosed herein.

BRIEF DESCRIPTION OF THE DRAWINGS

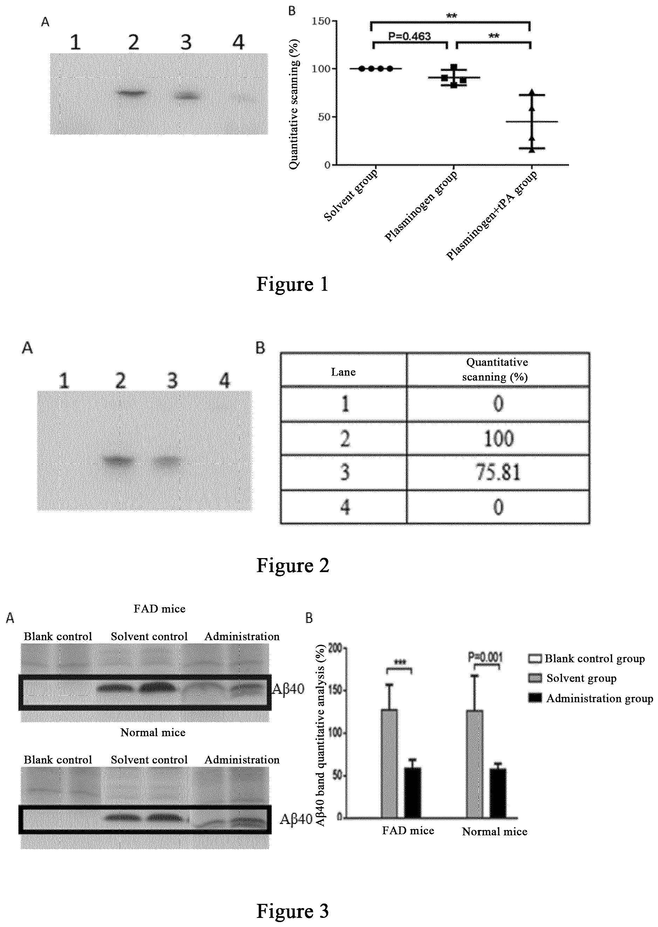

Fig. 1A and Fig. 1B show Tricine-SDS electrophoresis results of amyloid beta-protein 40 (Aβ40) dissolved in plasminogen in a PBS system. A shows a Tricine-SDS-PAGE electrophoretogram, and B shows quantitative scanning analysis results of Aβ40 dissolved in vitro. The results show that the Aβ40 content of a solvent control group is defined as 100% and does not have any change; Aβ40 of a plasminogen group is partially degraded in a case that plasminogen is added alone; and Aβ40 of a plasminogen+tPA group is obviously degraded in vitro in a case that plasminogen and tPA are added, and the difference between the plasminogen+tPA group and the solvent control group is significant (** indicates P<0.01). It indicates that plasminogen can promote the degradation of Aβ40.

Fig. 2A and Fig. 2B show Tricine-SDS electrophoresis results of Aβ40 dissolved in plasminogen in cerebrospinal fluids of rabbits, wherein line 1 and line 4 show a blank control group; line 2 shows a solvent group; and line 3 shows a plasminogen group. A shows a Tricine-SDS-PAGE electrophoretogram, and B shows quantitative scanning analysis results of dissolved Aβ40. The results show that the Aβ40 content of the solvent control group is defined as 100% and does not have any change; and Aβ40 of the plasminogen group is partially degraded in a case that plasminogen is added alone and is degraded to 74.81%. It indicates that plasminogen can promote the degradation of Aβ40.

Fig. 3A and Fig. 3B show effects of plasminogen on human Aβ40 in cerebrospinal fluids. A shows Tricine-SDS-PAGE electrophoretograms, and B shows quantitative scanning analysis results of dissolved Aβ40. The results show that the Aβ40 content of a solvent control group is defined as 100% and does not have any change; and Aβ40 of a plasminogen group is partially degraded in a case that plasminogen is added alone and is degraded to 74.81%. It indicates that plasminogen can promote the degradation of human Aβ40 in the cerebrospinal fluids.

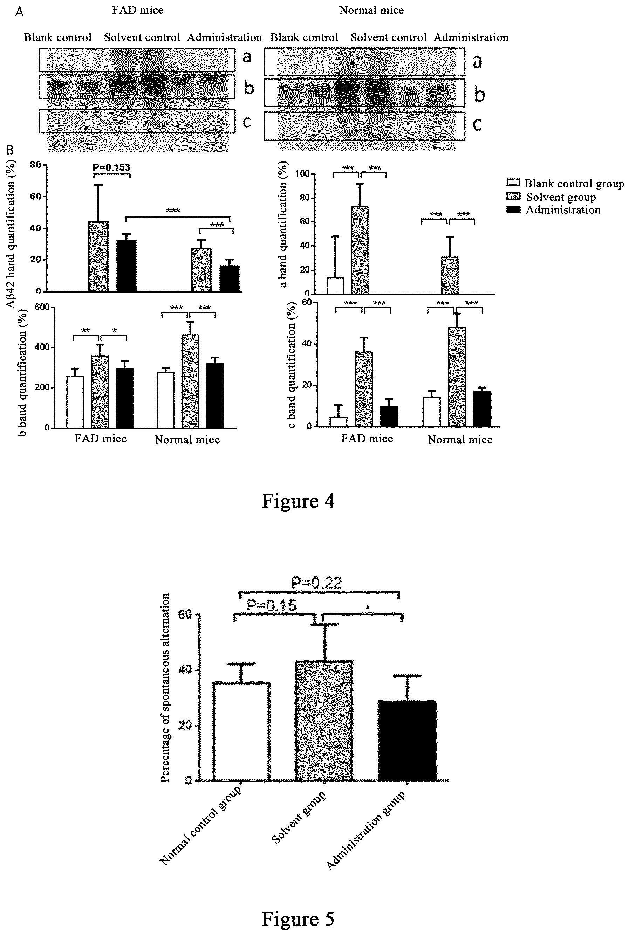

Fig. 4A and Fig. 4B show effects of plasminogen on human Aβ40 in cerebral homogenates of mouse models of Alzheimer's disease and normal mice. A shows Tricine-SDS-PAGE electrophoretograms, and B shows quantitative scanning analysis results of Aβ40 dissolved in vitro. The results show that in cerebral homogenates of the FAD mice, the Aβ40 content in a mouse of an administration group is obviously less than that in a mouse of a solvent control group, and the difference is extremely significant (*** indicates P<0.001); in the cerebral homogenates of the normal mice, the human Aβ40 content in a mouse of an administration group is obviously less than that in a mouse of a solvent control group, and the difference is extremely significant (P=0.001). It indicates that plasminogen can effectively promote the degradation of human Aβ40 in the cerebral homogenates of the mouse models of Alzheimer's disease and the normal mice.

Fig. 5 shows statistical results of the percentage of spontaneous alternation of a mouse model of Alzheimer's disease after 5 days of plasminogen administration. The results show that compared with a mouse of a normal control group, the percentage of spontaneous alternation of a mouse of a solvent group is obviously increased; the percentage of spontaneous alternation of a mouse of an administration group is obviously less than that of the mouse of the solvent control group, the statistical difference is significant (* indicates P<0.05), and the percentage of spontaneous alternation of the mouse of the administration group is closer to that of the mouse of the normal control group.

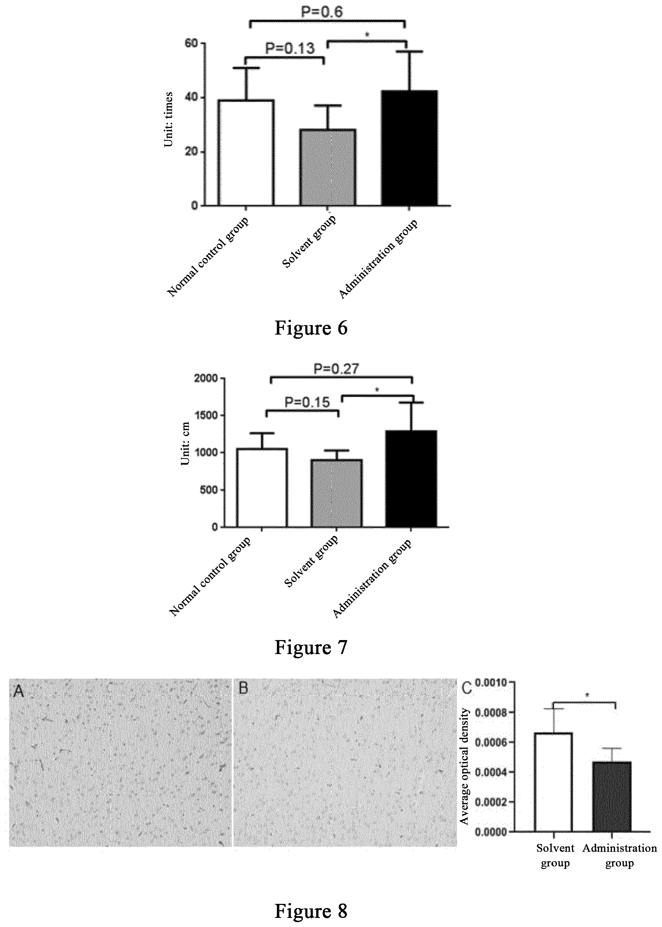

Fig. 6 shows statistical results of the total number of arm entries of a mouse model of Alzheimer's disease after 5 days of plasminogen administration. The results show that compared with a mouse of a normal control group, the total number of arm entries of a mouse of a solvent group is obviously decreased; the total number of arm entries of a mouse of an administration group is obviously greater than that of the mouse of the solvent control group, the statistical difference is significant (* indicates P<0.05), and the total number of arm entries of the mouse of the administration group is closer to that of the mouse of the normal control group.

Fig. 7 shows statistical results of a total travel distance of a mouse model of Alzheimer's disease after 5 days of plasminogen administration. The results show that compared with a mouse of a normal control group, a total travel distance of a mouse of a solvent group is obviously reduced; a total travel distance of a mouse of a plasminogen administration group is obviously longer than that of the mouse of the solvent control group, the statistical difference is significant (* indicates P<0.05), and the total travel distance of the mouse of the plasminogen administration group is closer to that of the mouse of the normal control group.

Fig. 8A to Fig. 8C show quantitative analysis results of stained Aβ42 in the cerebral cortex of a mouse model of Alzheimer's disease after 28 days of plasminogen administration. A shows a solvent group, B shows an administration group, and C shows quantitative analysis results of the average optical density. The results show that the level of Aβ42 deposition in the cerebral cortex of a mouse of the solvent group is obviously higher than that of a mouse of the administration group, and the statistical difference of the optical density quantitative analysis results is significant (* indicates P<0.05). It indicates that plasminogen can reduce Aβ42 deposition in the cerebral cortex of the mouse model of Alzheimer's disease.

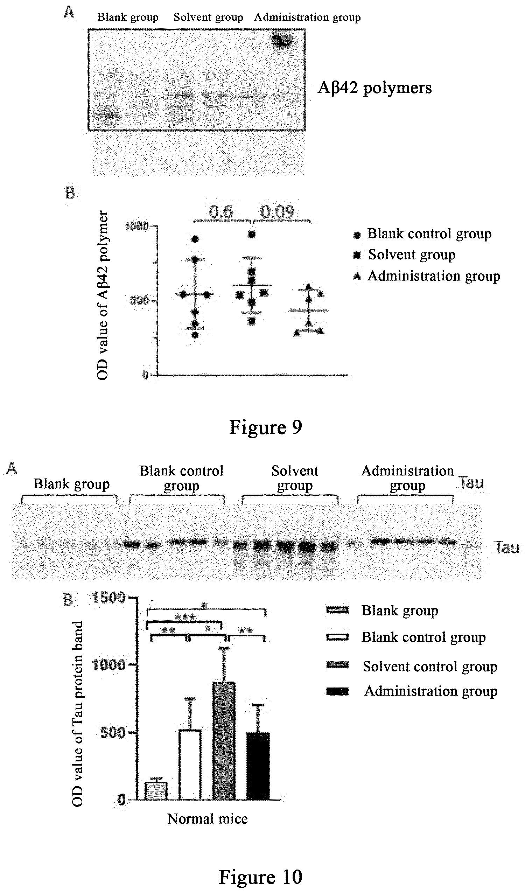

Fig. 9A and Fig. 9B show Western blot results of Aβ42 in a cerebral homogenate of a mouse model of Alzheimer's disease after 8 days of plasminogen administration. A shows a Western blot representative image, and B show quantitative analysis results of the optical density. The results show that a certain level of Aβ42 is present in a cerebral homogenate of a mouse of a blank control group; the Aβ42 level in a cerebral homogenate of a mouse of a solvent group is obviously higher than that of a mouse of an administration group, and the statistical P value is equal to 0.09. It indicates that plasminogen can reduce the Aβ42 level in the cerebral homogenate of the mouse model of Alzheimer's disease.

Fig. 10A and Fig. 10B show effects of plasminogen on Tau proteins in a cerebral homogenate of a normal mouse. A shows a Western blot image, and B shows quantitative analysis results of optical densities of Tau protein bands. The results show that in cerebral homogenates of normal mice, the Tau protein content in a mouse of a plasminogen group is obviously less than that in a mouse of a solvent control group, and the difference is significant (* indicates P<0.05, ** indicates P<0.01, and *** indicates P<0.001). It indicates that plasminogen can promote the degradation of Tau proteins in the cerebral homogenate of the normal mouse.

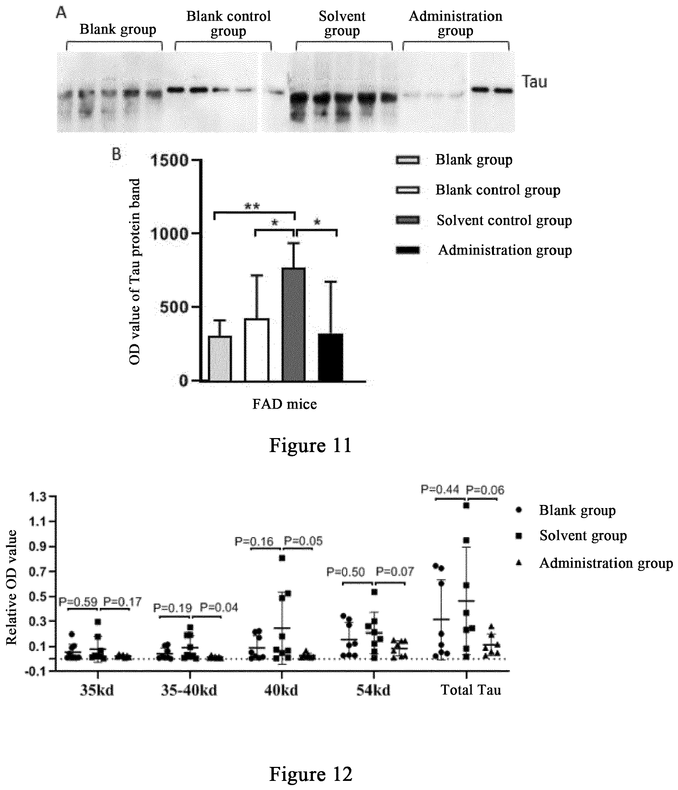

Fig. 11A and Fig. 11B show effects of plasminogen on Tau proteins in a cerebral homogenate of a mouse model of Alzheimer's disease. A shows a Western blot image, and B shows quantitative analysis results of optical densities of Tau protein bands. The results show that in cerebral homogenates of mouse models of Alzheimer's disease, the Tau protein content in a mouse of a plasminogen group is obviously less than that in a mouse of a solvent control group, and the statistical difference is significant (* indicates P<0.05, and ** indicates P<0.01). It indicates that plasminogen can promote the degradation of Tau proteins in the cerebral homogenate of the mouse model of Alzheimer's disease.

Fig. 12 shows Western blot assay results of Tau proteins having different molecular weights in brain tissue of a mouse model of Alzheimer's disease after 28 days of plasminogen administration. The results show that certain levels of Tau proteins having different molecular weights are present in a cerebral homogenate of a mouse of a blank control group; levels of Tau proteins having different molecular weights and the total Tau protein level in brain tissue of a mouse of an administration group are obviously lower than those of a mouse of a solvent group, and statistical analysis P values between the two groups in the levels of Tau proteins having molecular weights of 35 kd, 35-40 kd, 40 kd, and 54 kd, and the total Tau protein level are 0.174, 0.0406, 0.052, 0.067, and 0.055, respectively. It indicates that plasminogen can promote the degradation of Tau proteins in brain tissue of the mouse model of Alzheimer's disease.

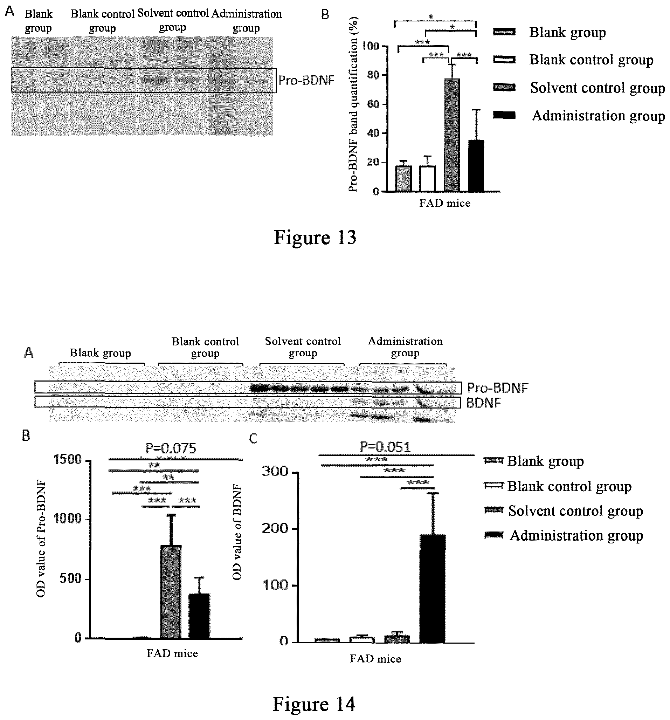

Fig. 13A and Fig. 13B show effects of plasminogen on recombinant human Pro-BDNF in a cerebral homogenate of a mouse model of Alzheimer's disease. A shows an SDS-PAGE electrophoregram, and B shows quantitative analysis results of Pro-BDNF bands in the SDS-PAGE electrophoregram. The results show that in cerebral homogenates of mouse models of Alzheimer's disease, the Pro-BDNF content in a mouse of a plasminogen administration group is obviously less than that in a mouse of a solvent control group, and the difference is extremely significant (* indicates P<0.05, and *** indicates P<0.001). It indicates that plasminogen can promote the cleavage of Pro-BDNF in the cerebral homogenate of the mouse model of Alzheimer's disease.

Fig. 14A to Fig. 14C show effects of plasminogen on recombinant human Pro-BDNF in a cerebral homogenate of a mouse model of Alzheimer's disease, wherein A shows a Western blot image, B shows analysis results of optical density (OD) values of Pro-BDNF bands in the Western blot image, and C shows analysis results of optical density (OD) values of BDNF bands in the Western blot image. The results show that in cerebral homogenates of mouse models of Alzheimer's disease, the Pro-BDNF content in a mouse of a plasminogen administration group is obviously less than that in a mouse of a solvent control group, and the difference is extremely significant (** indicates P<0.01, and *** indicates P<0.001); the BDNF content in the mouse of the plasminogen administration group is obviously greater than that in the mouse of the solvent control group, and the difference is extremely significant. It indicates that plasminogen can promote the cleavage of Pro-BDNF and formation of mature BDNF in the cerebral homogenate of the mouse model of Alzheimer's disease.

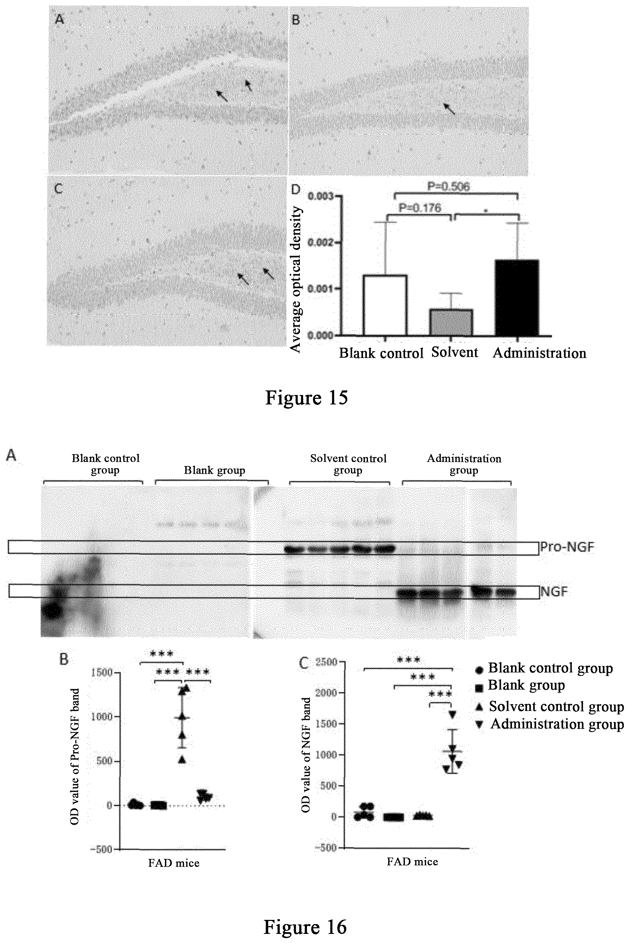

Fig. 15A to Fig. 15D show immumohistochemical staining results of BDNF in the hippocampus of a mouse model of Alzheimer's disease after 28 days of plasminogen administration. A shows a blank control group, B shows a solvent group, C shows an administration group, and D shows quantitative analysis results of the average optical density. The results show that a certain level of BDNF (indicated by arrows) is expressed in the hippocampus of a mouse of the blank control group; the expression of BDNF in the hippocampus of a mouse of the solvent group is obviously lower than that of the mouse of the blank control group; the expression of BDNF in the hippocampus of a mouse of the administration group is obviously greater than that of the mouse of the solvent group, and the statistical difference is significant (* indicates P<0.05). It indicates that plasminogen can promote the expression of BDNF in the hippocampus of the mouse model of Alzheimer's disease.

Fig. 16A to Fig. 16C show effects of plasminogen on recombinant human Pro-NGF in a cerebral homogenate of a mouse model of Alzheimer's disease. A shows a Western blot image, B shows analysis results of optical density (OD) values of Pro-NGF bands in the Western blot image, and C shows analysis results of optical density (OD) values of NGF bands in the Western blot image. The results show that in cerebral homogenates of mouse models of Alzheimer's disease, the Pro-NGF content in a mouse of a plasminogen administration group is obviously less than that in a mouse of a solvent control group, and the difference is extremely significant (*** indicates P<0.001); the NGF content in the mouse of the plasminogen administration group is obviously greater than that in the mouse of the solvent control group, and the difference is significant. It indicates that plasminogen can promote the cleavage of recombinant human Pro-NGF and formation of mature NGF in the cerebral homogenate of the mouse model of Alzheimer's disease.

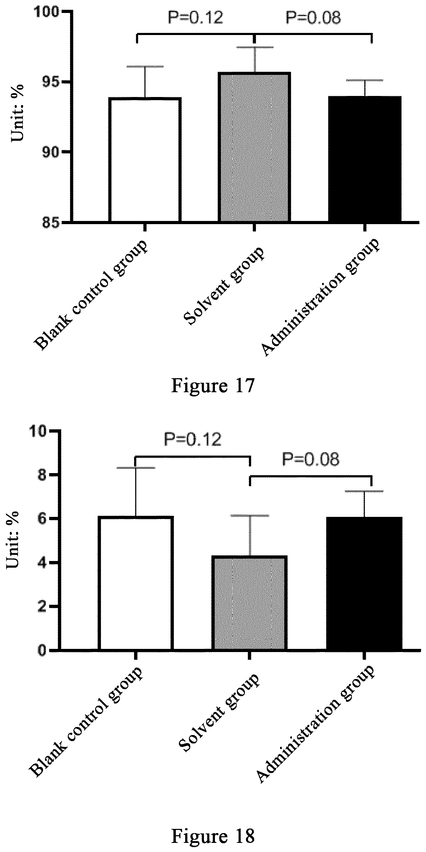

Fig. 17 shows statistical results of the percentage of boundary zone travel distance of a mouse model of Alzheimer's disease in an open field test after 28+7 days of plasminogen administration. The results show that a mouse of a blank control group has certain percentage of boundary zone travel distance; the percentage of boundary zone travel distance of a mouse of a solvent group is obviously greater than that of the mouse of the blank control group; the percentage of boundary zone travel distance of a mouse of an administration group is obviously less than that of the mouse of the solvent group, and the statistical difference is close to significant (P=0.08). It indicates that plasminogen can promote the recovery of anxiety and depression behaviors of the mouse model of Alzheimer's disease.

Fig. 18 shows statistical results the percentage of central zone travel distance of a mouse model of Alzheimer's disease in an open field test after 28+7 days of plasminogen administration. The results show that a mouse of a blank control group has certain percentage central zone travel distance; the percentage of central zone travel distance of a mouse of a solvent group is obviously less than that of the mouse of the blank control group; the percentage of central zone travel distance of a mouse of an administration group is obviously greater than that of the mouse of the solvent group, and the statistical difference is close to significant (P=0.08). It indicates that plasminogen can promote the recovery of anxiety and depression behaviors of the mouse model of Alzheimer's disease.

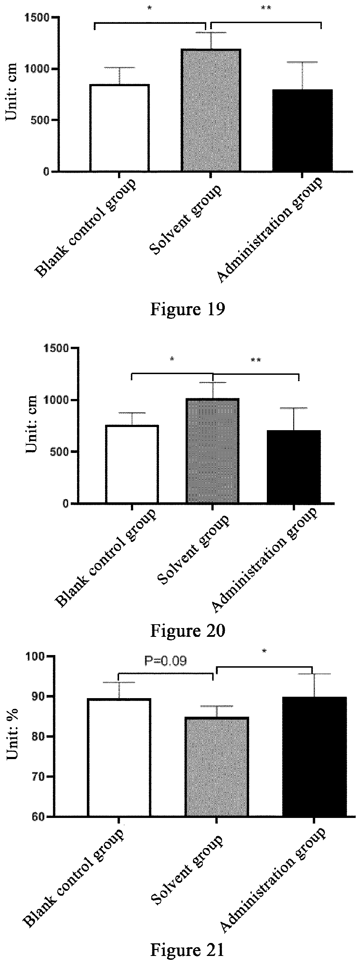

Fig. 19 shows statistical results of a total travel distance of a mouse model of Alzheimer's disease in an elevated plus maze test after 28+9 days of plasminogen administration. The results show that a mouse of a blank control group has a certain total travel distance; a total travel distance of a mouse of a solvent group is longer than that of the mouse of the blank control group; a total travel distance of a mouse of an administration group is shorter than that of the mouse of the solvent group, the statistical difference is extremely significant (* indicates P<0.05, and ** indicates P<0.01), and the total travel distance of the mouse of the administration group is closer to that of the mouse of the blank control group. It indicates that plasminogen can promote the recovery of anxiety and depression behaviors of the mouse model of Alzheimer's disease.

Fig. 20 shows statistical results of a closed arm travel distance of a mouse model of Alzheimer's disease in an elevated plus maze test after 28+9 days of plasminogen administration. The results show that a mouse of a blank control group has a certain closed arm travel distance; a closed arm travel distance of a mouse of a solvent group is obviously longer than that of the mouse of the blank control group; a closed arm travel distance of a mouse of an administration group is obviously shorter than that of the mouse of the solvent group, the statistical difference between the two groups is significant (* indicates P<0.05, and ** indicates P<0.01), and the closed arm travel distance of the mouse of the administration group is closer to that of the mouse of the blank control group. It indicates that plasminogen can promote the recovery of anxiety and depression behaviors of the mouse model of Alzheimer's disease.

Fig. 21 shows statistical results of the percentage of closed arm travel distance of a mouse model of Alzheimer's disease in an elevated plus maze test after 28+9 days of plasminogen administration. The results show that a mouse of a blank control group has certain percentage of closed arm travel distance; the percentage of closed arm travel distance of a mouse of a solvent group is obviously greater than that of the mouse of the blank control group; the percentage of closed arm travel distance of a mouse of an administration group is obviously less than that of the mouse of the solvent group, the statistical difference between the two groups is significant (* indicates P<0.05), and the percentage of closed arm travel distance of the mouse of the administration group is closer to that of the mouse of the blank control group. It indicates that plasminogen can promote the recovery of anxiety and depression behaviors of the mouse model of Alzheimer's disease.

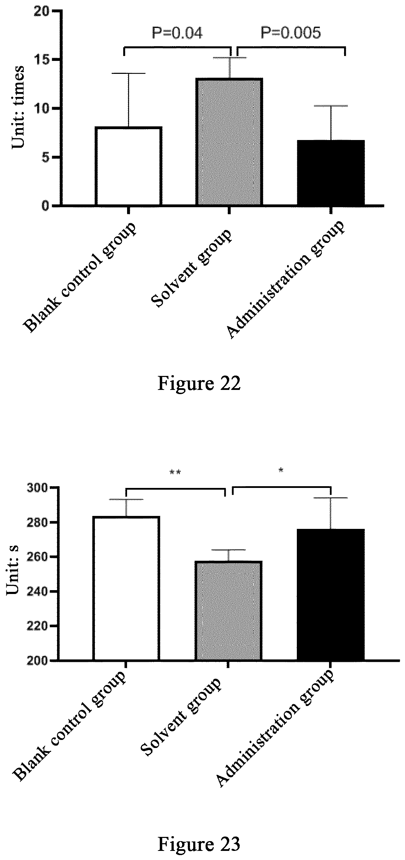

Fig. 22 shows statistical results of the number of closed arm entries of a mouse model of Alzheimer's disease in an elevated plus maze test after 28+9 days of plasminogen administration. The results show that a mouse of a blank control group has certain number of closed arm entries; the number of closed arm entries of a mouse of a solvent group is obviously greater than that of the mouse of the blank control group; the number of closed arm entries of a mouse of an administration group is obviously less than that of the mouse of the solvent group, the statistical difference between the two groups is extremely significant (* indicates P<0.05, and ** indicates P<0.01), and the number of closed arm entries of the mouse of the administration group is closer to that of the mouse of the blank control group. It indicates that plasminogen can promote the recovery of anxiety and depression behaviors of the mouse model of Alzheimer's disease.

Fig. 23 shows statistical results of closed arm duration of a mouse model of Alzheimer's disease in an elevated plus maze test after 28+9 days of plasminogen administration. The results show that a mouse of a blank control group has certain closed arm duration; closed arm duration of a mouse of a solvent group is obviously shorter than that of the mouse of the blank control group; closed arm duration of a mouse of an administration group is obviously longer than that of the mouse of the solvent group, the statistical difference between the two groups is significant (* indicates P<0.05, and ** indicates P<0.01), and the closed arm duration of the mouse of the administration group is closer to that of the mouse of the blank control group.

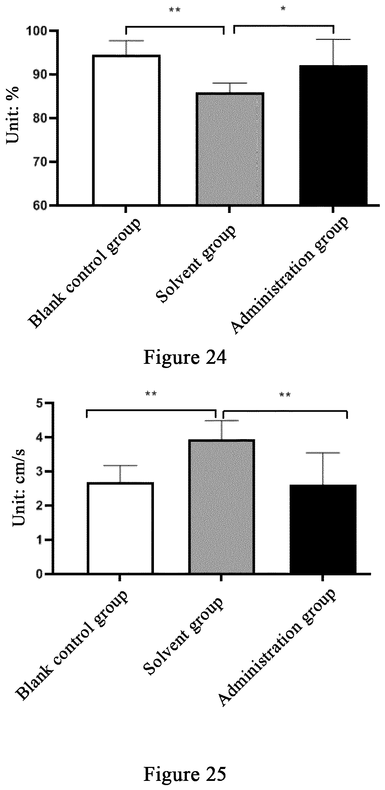

Fig. 24 shows statistical results of the percentage of closed arm duration of a mouse model of Alzheimer's disease in an elevated plus maze test after 28+9 days of plasminogen administration. The percentage of closed arm duration refers to a ratio of time spent by a mouse in a closed arm to total recording time. The results show that a mouse of a blank control group has certain percentage of closed arm duration; the percentage of closed arm duration of a mouse of a solvent group is obviously less than that of the mouse of the blank control group; the percentage of closed arm duration of a mouse of an administration group is obviously greater than that of the mouse of the solvent group, the statistical difference between the two groups is significant (* indicates P<0.05, and ** indicates P<0.01), and the percentage of closed arm duration of the mouse of the administration group is closer to that of the mouse of the blank control group.

Fig. 25 shows statistical results of a closed arm average movement speed of a mouse model of Alzheimer's disease in an elevated plus maze test after 28+9 days of plasminogen administration. The results show that a mouse of a blank control group has a certain closed arm average movement speed; a closed arm average movement speed of a mouse of a solvent group is higher than that of the mouse of the blank control group; a closed arm average movement speed of a mouse of an administration group is obviously lower than that of the mouse of the solvent group, the statistical difference between the two groups is extremely significant (** indicates P<0.01), and the closed arm average movement speed of the mouse of the administration group is closer to that of the mouse of the blank control group. It indicates that plasminogen can promote the recovery of anxiety and depression behaviors of the mouse model of Alzheimer's disease.

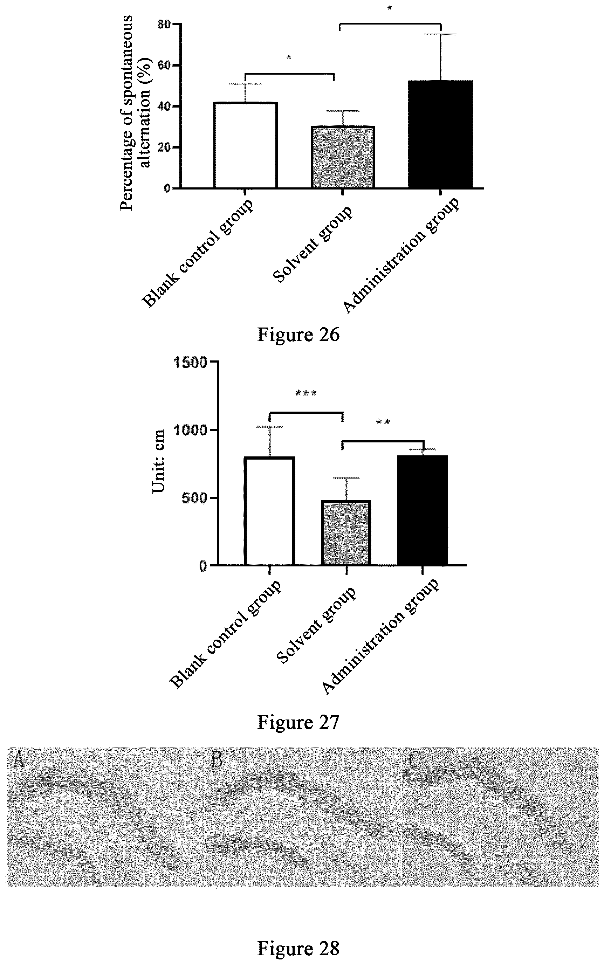

Fig. 26 shows statistical results of the percentage of spontaneous alternation of a mouse model of Alzheimer's disease in a Y maze test after 28+9 days of plasminogen administration. The results show that compared with a mouse of a blank control group, the percentage of spontaneous alternation of a mouse of a solvent group is obviously decreased; the percentage of spontaneous alternation of a mouse of an administration group is obviously greater than that of the mouse of the solvent group, the statistical difference between the two groups is significant (* indicates P<0.05), and the percentage of spontaneous alternation of the mouse of the administration group is closer to that of the mouse of the blank control group. It indicates that plasminogen can promote the recovery of memory function of the mouse model of Alzheimer's disease.

Fig. 27 shows statistical results of a closed arm travel distance of a mouse model of Alzheimer's disease in an elevated plus maze test after 18 days of plasminogen administration. The results show that a mouse of a blank control group has a certain closed arm travel distance; a closed arm travel distance of a mouse of a solvent group is obviously shorter than that of the mouse of the blank control group; a closed arm travel distance of a mouse of an administration group is obviously longer than that of the mouse of the solvent group, the statistical difference between the two groups is significant (** indicates P<0.01, and *** indicates P<0.001), and the closed arm travel distance of the mouse of the administration group is closer to that of the mouse of the blank control group. It indicates that plasminogen can promote the recovery of anxiety and depression behaviors of the mouse model of Alzheimer's disease.

Fig. 28A to Fig. 28C show representative images of HE staining of brain tissue of a mouse model of Alzheimer's disease after 8 days of plasminogen administration. A shows a blank control group, B shows a solvent control group, and C shows an administration group. The results show that the morphology of hippocampal tissue of a mouse of the blank control group is normal; and compared with the solvent group, the morphology of injured hippocampal tissue of a mouse of the administration group is obviously improved. It indicates that plasminogen can improve hippocampal damage in the mouse model of Alzheimer's disease.

DETAILED DESCRIPTION OF THE INVENTION

Fibrinolytic system is a system composed of a series of chemical substances involved in fibrinolysis. The chemical substances mainly include plasminogen, plasmin, plasminogen activators, and fibrinolysis inhibitors. The plasminogen activators include a tissue-type plasminogen activator (t-PA) and a urokinase-type plasminogen activator (u-PA). t-PA is a serine protease synthesized by vascular endothelial cells. t-PA activates plasminogen mainly on fibrin. The urokinase-type plasminogen activator (u-PA) is produced by renal tubular epithelial cells and vascular endothelial cells, and can directly activate plasminogen without the need for fibrin as a cofactor. Plasminogen (PLG) is synthesized in the liver. When blood coagulates, a large amount of PLG is adsorbed onto the fibrin network, and is activated to plasmin under the action of t-PA or u-PA to promote fibrinolysis. Plasmin (PL) is a serine protease, and has the following effects: degrading fibrin and fibrinogen; hydrolyzing a variety of blood coagulation factors such as V, VIII, X, VII, XI, and II; enabling plasminogen to be transformed into plasmin; hydrolyzing complements, etc. The fibrinolysis inhibitors include: plasminogen activator inhibitors (PAIs) and $\alpha$2 antiplasmin ($\alpha$2-AP). PAIs mainly include two types, i.e. PAI-1 and PAI-2, and can specifically bind to t-PA in a ratio of 1:1 to inactivate t-PA and activate PLG at the same time. $\alpha$2-AP is synthesized in the liver, and binds to PL in a ratio of 1: 1 to form a complex so as to inhibit the activity of PL. FXIII enables $\alpha$2-AP to bind to fibrin in the form of covalent bond to attenuate the sensitivity of fibrin to the action of PL. Substances inhibiting the activity of the fibrinolytic system in vivo include: PAI-1, a complement C1 inhibitor, $\alpha$2 antiplasmin, and an $\alpha$2 macroglobulin.

Herein, the term "component of a plasminogen activation pathway" covers:

1. plasminogen, Lys-plasminogen, Glu-plasminogen, micro-plasminogen, delta-plasminogen, and variants and analogs thereof;
2. plasmin and variants and analogs thereof; and
3. plasminogen activators, such as tPA, uPA, and a tPA or uPA variant or analog containing one or more domains (e.g. one or more kringle domains and proteolysis domains) of tPA or uPA.

The above "variants" of plasminogen, plasmin, tPA, and uPA include all naturally occurring human genetic variants and other mammalian forms of these proteins, and a protein that is obtained by adding, deleting and/or substituting, for example, 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid and still has the activity of plasminogen, plasmin, tPA or uPA. For example, the "variants" of plasminogen, plasmin, tPA, and uPA include mutational variants of these proteins that are obtained by substituting, for example, 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid with conservative amino acids.

The "plasminogen variants" of the present invention include proteins having at least 75%, 80%, 85%, 90%, 95%, 96%, 97%, 98% or 99% sequence identity with sequence 2, 6, 8, 10 or 12 and still having the activity, such as the lysine binding activity and the proteolytic activity, of plasminogen. For example, the "plasminogen variants" of the present invention may be proteins that are obtained by adding, deleting and/or substituting 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid on the basis of sequence 2, 6, 8, 10 or 12 and still have the activity, such as the lysine binding activity and the proteolytic activity, of plasminogen. Specifically, the plasminogen variants of the present invention include all naturally occurring human genetic variants and other mammalian forms of these proteins, and mutational variants of these proteins that are obtained by substituting, for example, 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid with conservative amino acids.

The plasminogen of the present invention may be a human plasminogen ortholog from a primate or a rodent, or a variant thereof that retains the activity, such as the lysine binding activity and the proteolytic activity, of plasminogen, such as plasminogen shown as sequence 2, 6, 8, 10 or 12, and human natural plasminogen shown as sequence 2.

The above "analogs" of plasminogen, plasmin, tPA, and uPA include compounds respectively providing functions basically similar to those of plasminogen, plasmin, tPA, or uPA.

The above "variants" and "analogs" of plasminogen, plasmin, tPA, and uPA include "variants" and "analogs" containing one or more domains (e.g. one or more kringle domains and proteolysis domains) of plasminogen, plasmin, tPA, and uPA. For example, the "variants" and "analogs" of plasminogen include plasminogen variants and analogs containing one or more domains (e.g. one or more kringle domains and proteolysis domains) of plasminogen, such as mini-plasminogen. The "variants" and "analogs" of plasmin include plasmin "variants" and "analogs" containing one or more domains (e.g. one or more kringle domains and proteolysis domains) of plasmin, such as mini-plasmin and delta-plasmin (δ-plasmin).

Whether the above "variants" or "analogs" of plasminogen, plasmin, tPA or uPA have the activity of plasminogen, plasmin, tPA or uPA, or whether the above "variants" or "analogs" of plasminogen, plasmin, tPA or uPA respectively provides functions basically similar to those of plasminogen, plasmin, tPA or uPA can be detected by the methods known in the art. For example, the activity of activated plasmin is determined by enzymography, an enzyme-linked immunosorbent assay (ELISA) or fluorescence-activated cell sorting (FACS), or determined by the methods described in the following documents: Ny, A., Leonardsson, G., Hagglund, A.C, Hagglof, P., Ploplis, V.A., Carmeliet, P. and Ny, T. (1999). Ovulation inplasminogen-deficient mice. Endocrinology 140, 5030-5035; Silverstein RL, Leung LL, Harpel PC, Nachman RL (November 1984). "Complex formation of platelet thrombospondin with plasminogen. Modulation of activation by tissue activator". J. Clin. Invest. 74 (5): 1625-33; Gravanis I, Tsirka SE (February 2008). "Tissue-type plasminogen activator as a therapeutic target in stroke". Expert Opinion on Therapeutic Targets. 12 (2): 159-70; and Geiger M, Huber K, Wojta J, Stingl L, Espana F, Griffin JH, Binder BR (Aug 1989). "Complex formation between urokinase and plasma protein C inhibitor in vitro and in vivo". Blood. 74 (2): 722-8.

In some embodiments of the present invention, the "component of the plasminogen activation pathway" of the present invention is plasminogen. In some embodiments, the plasminogen is human full-length plasminogen or a conservatively substituted variant thereof that retains the activity (e.g. the lysine binding activity and the proteolytic activity) of plasminogen. In some embodiments, the plasminogen is selected from Glu-plasminogen, Lys-plasminogen, mini-plasminogen, micro-plasminogen, delta-plasminogen, and variants thereof that retain the activity (e.g. the lysine binding activity and the proteolytic activity) of plasminogen. In some embodiments, the plasminogen is natural or synthesized human plasminogen, or a conservatively substituted variant or fragment thereof that retains the activity (e.g. the lysine binding activity and the proteolytic activity) of plasminogen. In some embodiments, the plasminogen is a human plasminogen ortholog from a primate or a rodent, or a conservatively substituted variant or fragment thereof that retains the activity of plasminogen. In some embodiments, the plasminogen contains an amino acid sequence shown as sequence 2, 6, 8, 10 or 12. In some embodiments, the plasminogen contains a conservatively substituted sequence of the amino acid sequence shown as sequence 2, 6, 8, 10 or 12. In some embodiments, the plasminogen has an amino acid sequence shown as sequence 2, 6, 8, 10 or 12. In some embodiments, the plasminogen is a conservatively substituted variant of plasminogen shown as sequence 2, 6, 8, 10 or 12. In some embodiments, the plasminogen is human natural plasminogen or a conservative mutant thereof. In some embodiments, the plasminogen is human natural plasminogen shown as sequence 2 or a conservatively substituted variant thereof.

A "compound capable of directly activating plasminogen or indirectly activating plasminogen by activating an upstream component of a plasminogen activation pathway" refers to any compound that can directly activate plasminogen or indirectly activate plasminogen by activating an upstream component of a plasminogen activation pathway, such as tPA, uPA, streptokinase, saruplase, alteplase, reteplase, tenecteplase, anistreplase, monteplase, lanoteplase, pamiteplase, and staphylokinase.

An "antagonist of a fibrinolysis inhibitor" of the present invention is a compound that antagonizes, weakens, blocks, or prevents the action of a fibrinolysis inhibitor. The fibrinolysis inhibitor is, for example, PAI-1, a complement C1 inhibitor, $\alpha2$ antiplasmin or an $\alpha2$ macroglobulin. The antagonist is, for example, an antibody of PAI-1, a complement C1 inhibitor, $\alpha2$ antiplasmin or an $\alpha2$ macroglobulin, or antisense RNA or small RNA that blocks or down-regulates the expression of PAI-1, a complement C1 inhibitor, $\alpha2$ antiplasmin or an $\alpha2$ macroglobulin, or a compound that occupies a binding site of PAI-1, a complement C1 inhibitor, $\alpha2$ antiplasmin or an $\alpha2$ macroglobulin and does not have functions of PAI-1, a complement C1 inhibitor, $\alpha2$ antiplasmin or an $\alpha2$ macroglobulin, or compound that blocks a binding domain and/or an activity domain of PAI-1, a complement C1 inhibitor, $\alpha2$ antiplasmin or an $\alpha2$ macroglobulin.

Plasmin is a key component of a plasminogen activation system. It is a broad-spectrum protease, and can hydrolyze several components, including fibrin, gelatin, fibronectin, laminin, and proteoglycans, of an extracellular matrix (ECM). In addition, plasmin can activate some matrix metalloproteinase precursors (pro-MMPs) to active matrix metalloproteinases (MMPs) Therefore, plasmin is considered as an important upstream regulator of extracellular proteolysis. Plasmin is formed by proteolysis of plasminogen with two types of physiological PAs, i.e. a tissue-type plasminogen activator (tPA) and a urokinase-type plasminogen activator (uPA). Due to relatively high levels of plasmin in plasma and other body fluids, it has traditionally been thought that the regulation of the PA system is mainly achieved through the synthesis and activity levels of PAs. The synthesis of components of the PA system is strictly regulated by different factors, such as a hormone, a growth factor, and a cytokine. In addition, there are specific physiological inhibitors of plasmin and PAs. A main inhibitor of plasmin is $\alpha2$-antiplasmin. The activity of PAs is regulated by both of a plasminogen activator inhibitor 1 (PAI-1) for inhibiting uPA and tPA and a plasminogen activator inhibitor 2 (PAI-2) for mainly inhibiting uPA. There are uPA-specific cell surface receptors (uPARs) having the direct hydrolysis activity on the surface of some cells.

Plasminogen is a single-stranded glycoprotein, is composed of 791 amino acids, and has a molecular wight of about 92 kDa. Plasminogen is mainly synthesized in the liver, and is abundant in the extracellular fluid. The plasminogen content in plasma is about 2 μM. Therefore, plasminogen is a huge potential source of the proteolytic activity in tissues and body fluids. Plasminogen is present in two molecular forms, i.e. glutamate-plasminogen (Glu-plasminogen) and lysine-plasminogen (Lys-plasminogen). Naturally secreted and uncleaved plasminogen has an amino-terminal (N-terminal) glutamate, so it is referred to as glutamate-plasminogen. However, glutamate-plasminogen is hydrolyzed to lysine-plasminogen at Lys76-Lys77 in the presence of plasmin. Compared with glutamate-plasminogen, lysine-plasminogen has higher affinity to fibrin and can be activated by PAs at a higher rate. Arg560-Val561 peptide bonds of the two forms of plasminogen can be cleaved by uPA or tPA to form double-stranded protease plasmin linked via a disulfide bond. The amino-terminal moiety of plasminogen contains five homologous tri-circles, i.e. kringles; and the carboxyl-terminal moiety of plasminogen contains protease domains. Some kringles contain lysine binding sites for mediating specific interaction of plasminogen and fibrin, as well as its inhibitor $\alpha2$-AP. It is found recently that a plasminogen fragment of 38 kDa that contains kringles 1 to 4 is an effective inhibitor for angiogenesis. This fragment is named angiostatin, which can be produced by hydrolyzing plasminogen with several proteases.

A main substrate of plasmin is fibrin, and the dissolution of fibrin is a key for preventing pathological thrombosis. Plasmin also has substrate specificity to several components, including laminin, fibronectin, proteoglycans, and gelatin, of ECM, suggesting that plasmin also plays an important role in reconstruction of ECM. Indirectly, plasmin can also degrade other components, including MMP-1, MMP-2, MMP-3, and MMP-9, of ECM by transforming some protease precursors into active proteases. Therefore, it has been proposed that plasmin may be an important upstream regulator of extracellular proteolysis. In addition, plasmin has the ability to activate certain potential growth factors. In vitro, plasmin can also hydrolyze components of a complement system and release chemotactic complement fragments.

"Plasmin" is a very important enzyme present in the blood, which can hydrolyze a fibrin clot to fibrin degradation products and D-dimer.

"Plasminogen" is the zymogen form of plasmin. According to sequences in Swiss Prot, a glycoprotein composed 810 amino acids, having a molecular weight of about 90 kDa, mainly synthesized in the liver, and capable of circulating in the blood is calculated based on an amino acid sequence (sequence 4) of natural human plasminogen containing a signal peptide, and a cDNA sequence for encoding the amino acid sequence is shown as sequence 3. Full-length plasminogen contains seven domains, i.e. a serine protease domain at the C terminus, a Pan Apple (PAp) domain at the N terminus, and five Kringle domains (Kringle1 to Kringle5). Referring to sequences in Swiss Prot, the signal peptide includes residues Met1-Gly19, PAp includes residues Glu20-Val98, Kringle1 includes residues Cys103-Cys181, Kringle2 includes residues Glu184-Cys262, Kringle3 includes residues Cys275-Cys352, Kringle4 includes residues Cys377-Cys454, and Kringle5 includes residues Cys481-Cys560. According to data of NCBI, the serine protease domain includes residues Val581-Arg804.

Glu-plasminogen is human natural full-length plasminogen and is composed of 791 amino acids (without a signal peptide of 19 amino acids), a cDNA sequence for encoding the sequence is shown as sequence 1, and an amino acid sequence of Glu-plasminogen is shown as sequence 2. In vivo, there is Lys-plasminogen formed by hydrolyzing Glu-plasminogen at amino acids at the 76th site and the 77th site, which is shown as sequence 6, and a cDNA sequence for encoding the amino acid sequence is shown as sequence 5. Delta-plasminogen (δ-plasminogen) is full-length plasminogen lacking a fragment from Kringle2 to Kringle5 and containing only Kringle1 and a serine protease domain (also referred to as a protease domain (PD)), an amino acid sequence (sequence 8) of delta-plasminogen has been reported in a document, and a cDNA sequence for encoding the amino acid sequence is shown as sequence 7. Mini-plasminogen is composed of Kringle5 and a serine protease domain, it has been reported in a document that an amino acid sequence of mini-plasminogen includes residues Val443-Asn791 (taking a Glu residue in a sequence of Glu-plasminogen without a signal peptide as the starting amino acid), and is shown as sequence 10, and a cDNA sequence for encoding the amino acid sequence is shown as sequence 9. Micro-plasminogen contains only a serine protease domain, it has been reported in a document that an amino acid sequence of micro-plasminogen includes residues Ala543-Asn791 (taking a Glu residue in a sequence of Glu-plasminogen without a signal peptide as the starting amino acid), and it has also been reported in the patent document CN102154253A that the sequence of micro-plasminogen includes residues Lys531-Asn791 (taking a Glu residue in a sequence of Glu-plasminogen without a signal peptide as the starting amino acid). In this patent application, the amino acid sequence of micro-plasminogen is referred to the patent document CN102154253A, and is shown as sequence 12, and a cDNA sequence for encoding the amino acid sequence is shown as sequence 11.

The structure of full-length plasminogen is also described in the paper of Aisina, et al. (Aisina R B, Mukhametova L I. Structure and function of plasminogen/plasmin system [J]. Russian Journal of Bioorganic Chemistry, 2014, 40 (6): 590-605). In this paper, Aisina, et al. describe that plasminogen includes Kringle1, 2, 3, 4, and 5 domains and a serine protease domain (also referred to as a protease domain (PD)). Kringles are responsible for binding plasminogen to ligands having low molecular wights and high molecular weights (i.e. the lysine binding activity), so that plasminogen is transformed into a more open conformation, which can be activated more easily. The protease domain (PD) includes residues Val562-Asn791, and tPA and uPA specifically cleave an activation bond at sites Arg561-Val562 in plasminogen to transform plasminogen into plasmin. Therefore, the protease domain (PD) is a region giving the proteolytic activity of plasminogen.

Herein, the terms "plasmin", "fibrinolysin", and "fibrinolytic enzyme" are interchangeable and have the same meaning. The terms "plasminogen", "profibrinolysin", and "fibrinolytic zymogen" are interchangeable and have the same meaning.

In the present invention, the "plasminogen deficiency" means that the plasminogen content or activity in a subject is less than that in a normal person, and is low enough to affect normal physiological functions of the subject. The "plasminogen deficiency" means that the plasminogen content or activity in a subject is less than that in a normal person, the activity or expression of plasminogen is extremely low, and normal physiological functions can only be maintained by providing exogenous plasminogen.

Those in the art may understand that all technical solutions of plasminogen of the present invention are applicable to plasmin, and thus the technical solutions described in the present invention cover plasminogen and plasmin. During circulation, plasminogen is in a closed inactive conformation, and is transformed into active plasmin in an open conformation under the mediation of a plasminogen activator (PA) when binding to a thrombus or cell surface. Active plasmin can further hydrolyze a fibrin clot to fibrin degradation products and D-dimer, so as to dissolve a thrombus. The PAp domain of plasminogen contains an important determinant for maintaining plasminogen in a closed inactive conformation, and the KR domain of plasminogen can bind to a lysine residue present in a receptor and a substrate. A variety of known enzymes that can be used as plasminogen activators include: a tissue-type plasminogen activator (tPA), a urokinase-type plasminogen activator (uPA), a kallikrein, a blood coagulation factor XII (Hageman factor), etc.

A "plasminogen active fragment" refers to a fragment having the activity of binding to lysine in a target sequence of a substrate (the lysine binding activity), or the activity of exerting proteolytic function (the proteolytic activity), or the proteolytic activity and the lysine binding activity. The technical solutions related to plasminogen of the present invention cover a technical solution of replacing plasminogen with a plasminogen active fragment. In some embodiments, the plasminogen active fragment of the present invention contains the serine protease domain of plasminogen or is composed of the serine protease domain of plasminogen. In some embodiments, the plasminogen active fragment of the present invention contains sequence 14, or contains an amino acid sequence having at least 80%, 90%, 95%, 96%, 97%, 98% or 99% identity with sequence 14, or is composed of sequence 14, or is composed of the amino acid sequence having at least 80%, 90%, 95%, 96%, 97%, 98% or 99% identity with sequence 14. In some embodiments, the plasminogen active fragment of the present invention contains one or more regions selected from Kringle1, Kringle2, Kringle3, Kringle4, and Kringle5 or conservatively substituted variants thereof, or is composed of one or more regions selected from Kringle1, Kringle2, Kringle3, Kringle4, and Kringle5 or conservatively substituted variants thereof. In some embodiments, the plasminogen of the present invention includes a protein containing the above plasminogen active fragment.

At present, assays of plasminogen in the blood and its activity include: an tissue-type plasminogen activator activity assay (t-PAA), a plasma tissue-type plasminogen activator antigen assay (t-PAAg), a plasma tissue-type plasminogen activity assay (plgA), a plasma tissue plasminogen antigen assay (plgAg), a plasma tissue-type plasminogen activator inhibitor activity assay, a plasma tissue-type plasminogen activator inhibitor antigen assay, and a plasma plasmin-antiplasmin complex assay (PAP). The most commonly used test method is a chromogenic substrate method: streptokinase (SK) and a chromogenic substrate are added to plasma to be tested, PLG in the plasma to be tested is transformed into PLM under the action of SK, PLM acts on the chromogenic substrate, absorbance is measured by using a spectrophotometer, and increase in absorbance is proportional to the activity of plasminogen. In addition, immunohistochemistry, gel electrophoresis, immunoturbidimetry, radial immunodiffusion, etc. can also be adopted to test the activity of plasminogen in the blood.

An "ortholog" refers to a homolog of different species, includes a protein homolog and a DNA homolog, and is also referred to as a vertical homolog. It specifically refers to a protein or a gene in different species that has evolved from the same ancestral gene. The plasminogen of the present invention includes human natural plasminogen, and also includes plasminogen orthologs derived from different species and having the activity of plasminogen.

A "conservatively substituted variant" refers to that a given amino acid residue is changed, but the whole conformation and function of a protein or enzyme are not changed. For example, an amino acid in an amino acid sequence of a parent protein is substituted with an amino acid with similar properties (e.g. acidity, alkalinity, and hydrophobicity). The amino acid with similar properties is well known. For example, arginine, histidine, and lysine are hydrophilic alkaline amino acids and can be substituted with each other. Similarly, isoleucine is a hydrophobic amino acid and can be substituted with leucine, methionine or valine. Therefore, the similarity of amino acid sequences of two protein having similar functions may be different. For example, 70% to 99% similarity (identity) based on the MEGALIGN algorithm. The "conservatively substituted variant" also includes a polypeptide or an enzyme that has more than 60% amino acid sequence identity determined based on BLAST or FASTA algorithm, preferably, more than 75% identity, more preferably, more than 85% identity, and the most preferably, more than 90% identity. The polypeptide or the enzyme has the same or basically similar properties or function compared to a natural or parent protein or enzyme.

"Isolated" plasminogen refers to a plasminogen protein isolated and/or recovered from a natural environment of plasminogen. In some embodiments, the plasminogen is purified (1) to the purity (by weight) of more than 90%, more than 95% or more than 98%, such as more than 99% determined by the Lowry method, (2) to an extent sufficient to obtain at least 15 residues at the N terminus or in an internal amino acid sequence by using a rotary cup sequencer, or (3) to homogeneity that is determined by sodium dodecyl sulfate-polyacrylamide gel electrophoresis (SDS-PAGE) using Coomassie blue or silver staining under reducing or non-reducing conditions. The isolated plasminogen also includes plasminogen prepared from a recombinant cell by a bioengineering technology and isolated by at least one purification step.

Herein, the terms "polypeptide", "peptide", and "protein" are interchangeable, refer to an aggregation form of amino acids of any length, and may include genetically encoded and non-genetically encoded amino acids, chemically or biogeochemically modified or derived amino acids, and a polypeptide having a modified peptide backbone. The terms include fusion proteins, which include, but are not limited to, a fusion protein having an heterogenous amino acid sequence, a fusion having heterogenous and homologous leader sequences (having or without an N-terminal methionine residue), etc.

"Amino acid sequence identity percentage (%)" relative to a reference polypeptide sequence is defined as, after gaps have been introduced as necessary to achieve the maximum percentage sequence identity, and no conservative substitutions are considered as a part of the sequence identity, the percentage of amino acid residues, which are identical to amino acid residues in the reference polypeptide sequence, in a candidate sequence. Comparison for determining percentage amino acid sequence identity can be achieved in a variety of ways within the technical scope of the art. For example, software available to the public, such as BLAST, BLAST-2, ALIGN, and Megalign (DNASTAR), is adopted. Those skilled in the art can determine appropriate parameters used for comparing sequences, such as any algorithm needed to achieve the maximum comparison over the full lengths of sequences to be compared. However, for the purpose of the present invention, an amino acid sequence identity percentage value is generated by using the sequence comparison computer program ALIGN-2.

In a case that ALIGN-2 is adopted to compare amino acid sequences, % amino acid sequence identity of a given amino acid sequence A relative to a given amino acid sequence B (or may be expressed as that the given amino acid sequence A has or contains certain % amino acid sequence identity relative to, with, or to the given amino acid sequence B) is calculated as follows:

$$X/Y \times 100\%$$

where, X is the number of amino acid residues, identically matched with amino acid residues in B, in A that is determined by the sequence comparison program ALIGN-2, and Y is the total number of amino acid residues in B. It is to be understood that in a case that the length of the amino acid sequence A differs from that of the amino acid sequence B, % amino acid sequence identity of A relative to B is not equal to % amino acid sequence identity of B relative to A. Unless otherwise specifically described, all % amino acid sequence identity values used herein are obtained by using the ALIGN-2 computer program as described in the preceding paragraph.

As used herein, the term "treatment" refers to obtaining a desired pharmacological and/or physiological effect. The effect may be complete or partial prevention of occurrence and onset of a disease or its symptoms, partial or complete alleviation of a disease and/or its symptoms, and/or partial or complete cure of a disease and/or its symptoms, which includes: (a) prevention of occurrence or onset of a disease in a subject who may have a predisposition to the disease but has not been diagnosed with the disease; (b) inhibition of a disease, i.e. retardation of the formation of the disease; and (c) alleviation of a disease and/or its symptoms, i.e. subsidence or disappearance of the disease and/or its symptoms.

Herein, the terms "individual", "subject", and "patient" are interchangeable, and refer to a mammal, which includes, but is not limited to, murine (rats and mice), non-human primates, humans, dogs, cats, ungulates (e.g. horses, cattle, sheep, pigs, and goats), etc.

A "therapeutically effective amount" or "effective dose" refers to an amount of a component of a plasminogen activation pathway or its related compound (e.g. plasminogen) that is sufficient to prevent and/or treat a disease when administered to a mammal or other subjects to treat the disease. The "therapeutically effective amount" is changed with the component of the plasminogen activation pathway or its related compound (e.g. plasminogen) used, a disease of a subject to be treated and/or the severity of symptoms, age, and weight, etc.

Preparation of the plasminogen of the present invention

Plasminogen can be isolated from nature and purified for further therapeutic use, or can be synthesized by a standard chemical peptide synthesis technology. In a case that a polypeptide is synthesized chemically, plasminogen can be synthesized from a liquid phase or a solid phase. Solid-phase polypeptide synthesis (SPPS) (in which a C-terminal amino acid of a sequence is attached to an insoluble support, followed by sequential addition of the remaining amino acids in the sequence) is a method suitable for chemical synthesis of plasminogen. Various SPPS methods, such as Fmoc and Boc, can be used to synthesize plasminogen. The solid-phase synthesis technique is described in Barany, et al. Solid-Phase Peptide Synthesis; The Peptides: Analysis, Synthesis, Biology. Vol. 2: Special Methods in Peptide Synthesis, Part A, 3-284; Merrifield. Solid Phase Peptide Synthesis. I. The Synthesis of a Tetrapeptide. J. Am. Chem. Soc., 85: 2149-2156 (1963); Stewart, et al. Solid Phase Peptide Synthesis, 2nd ed. Pierce Chem. Co., Rockford, Ill. (1984); Ganesan A. 2006 Mini Rev. Med Chem. 6: 3-10; and Camarero JA, et al. 2005 Protein Pept Lett. 12: 723-8. In short, small insoluble porous beads are treated with a functional unit on which a peptide chain is built. After recirculation of coupling/deprotection, the attached solid-phase free N-terminal amine is coupled to a single N-protected amino acid unit. This unit is then deprotected to reveal a new N-terminal amine that can be attached to another amino acid. The peptide remains immobilized on the solid phase and then is cleaved.

Plasminogen of the present invention can be produced by a standard recombination method. For example, a nucleic acid for encoding plasminogen is inserted into an expression vector so as to be operably connected to a regulatory sequence in the expression vector. The expression regulatory sequence includes, but is not limited to, a promoter (e.g. a naturally related or heterogenous promoter), a signal sequence, an enhancer element, and a transcription termination sequence. The expression regulatory sequence may be a eukaryotic promoter in the vector, and the vector can transform or transfect eukaryotic host cells (e.g. COS or CHO cells). Once the vector is incorporated into a suitable host, the vector maintains the host under conditions suitable for high expression of a nucleotide sequence and collection and purification of plasminogen.

The suitable expression vector usually replicates in the host organism as an episome or an integrated part of the host chromosomal DNA. Normally, the expression vector contains a selectable marker (e.g. ampicillin resistance, hygromycin b resistance, tetracycline resistance, kanamycin resistance, and neomycin resistance) to facilitate detection of cells transformed with an exogenous desired DNA sequence.

Exemplary prokaryotic host cells that can be used to clone a polynucleotide for encoding a subject antibody include *Escherichia coli*. Other suitable microbial hosts include: *Bacillus* such as *Bacillus subtilis,* and other *Enterobacteriaceae* such as *Salmonella, Serratia,* and various *Pseudomonas* species. Expression vectors can also be generated in these prokaryotic hosts, and usually contain expression control sequences (origin of replication) compatible with the host cells. In addition, there are many known promoters, such as a lactose promoter system, a tryptophan (trp) promoter system, a beta-lactamase promoter system, and a promoter system from bacteriophage λ. The promoter usually controls expression optionally in a sequence of an operator gene, and has a ribosome binding site sequence for initiating and completing transcription and translation.

Other microorganisms, such as yeast, can also be used for expression. Exemplary suitable yeast host cells include yeast (e.g. *Saccharomyces cerevisiae* (*S. cerevisiae*)) and *Pichia,* and the suitable vector has an expression control sequence (e.g. a promoter), origin of replication, a terminator sequence, etc. according to the requirements. Typical promoters contain 3-phosphoglycerate kinase and other glycogenolysis enzymes. Inducible yeast promoters specially include promotes from alcohol dehydrogenase, hetero-cytochrome C, and enzymes responsible for using maltose and galactose.

In addition to microorganisms, mammalian cells (e.g. mammalian cells cultured in a cell culture medium in vitro) can also be used for expressing and generating the anti-Tau antibody (e.g. a polynucleotide for encoding a subject anti-Tau antibody) of the present invention. Referring to Winnacker, From Genes to Clones, VCH Publishers, N.Y., N.Y. (1987). Suitable mammal host cells include a CHO cell line, various Cos cell line, HeLa cells, a myeloma cell line, and transformed B cells or hybridoma. An expression vector used for these cells may include an expression control sequence such as origin of replication, a promoter, and an enhancer (Queen, et al. Immunol. Rev. 89: 49 (1986)), and a necessary processing information site such as a ribosome binding site, an RNA splicing site, a polyadenylation site, and a transcription terminator sequence. Exemplary suitable expression control sequences include derived promoters such as a white immunoglobulin gene, SV40, an adenovirus, a bovine papillomavirus, and a cytomegalovirus Referring to Co, et al. J. Immunol. 148: 1149 (1992).

Once the plasminogen of the present invention is synthesized (by the chemical or recombination method), the plasminogen of the present invention is purified in accordance with the standard procedure in the art, which includes ammonium sulfate precipitation, affinity column chromatography, high performance liquid chromatography (HPLC), gel electrophoresis, etc. The plasminogen is substantially pure, for example, at least about 80% to 85% pure, at least 85% to 90% pure, at least about 90% to 95% pure, 98% to 99% pure or purer. For example, the plasminogen does not contain contaminants such as cellular debris and macromolecules other than the target product.

Drug preparation

A therapeutic preparation is a lyophilized preparation or an aqueous solution formed by mixing a component of a plasminogen activation pathway or its related compound (e.g. plasminogen) with required purity with an optional pharmaceutical carrier, an excipient or a stabilizer (Remington's Pharmaceutical Sciences, 16th Edition, Osol, A. ed. (1980)). The acceptable carrier, excipient or stabilizer at a used dose and concentration is nontoxic to subjects, and include a buffer such as phosphates, citrates, and other organic acids; an antioxidant such as ascorbic acid and methionine; a preservative (e.g. octadecyl dimethyl benzyl ammonium chloride, hexanediamine chloride, benzalkonium chloride, benzethonium chloride, phenol, butanol, benzyl alcohol, alkyl parabens such as methyl and ethyl parabens, catechol, resorcinol, cyclohexanol, 3-pentanol, and m-cresol); a polypeptide with a low molecular weight (less than about 10 residues); a protein such as serum albumin, gelatin, and immunoglobulin; a hydrophilic polymer such as polyvinylpyrrolidone; an amino acid such as glycine, glutamine, asparagine, histidine, arginine, and lysine; monosaccharide, disaccharide, and other carbohydrates such as glucose, mannose, and dextrin; a chelant such as EDTA; saccharides such as sucrose, mannitol, fucose, and sorbitol; salt-forming counterions such as sodium; a metal complex (e.g. a zinc-protein complex); and/or a non-ionic surfactant such as TWEENTM, PLURONICSTM, and polyethylene glycol (PEG). A preferred lyophilized anti-VEGF antibody preparation is described in WO 97/04801, which is is incorporated herein by reference

The preparation of the present invention may also contain more than one active compound needed for treating a specific symptom, and preferably, the active compounds are complementary and do not have side effects on each other.

The plasminogen of the present invention can be encapsulated in a microcapsule prepared by techniques such as coacervation or interfacial polymerization, for example, can be placed in a colloidal drug delivery system (e.g. a liposome, an albumin microsphere, a microemulsion, nanoparticles, and a nanocapsule) or placed in hydroxymethylcellulose in a macroemulsion or a gel-microcapsule and a poly-(methyl methacrylate) microcapsule. These techniques are disclosed in Remington's Pharmaceutical Sciences 16th edition, Osol, A. Ed. (1980).

The component of the plasminogen activation pathway or its related compound (e.g. plasminogen) used for in vivo administration needs to be sterile. It can be easily achieved by filtration with a sterile filter before or after lyophilization and re-preparation.

The component of the plasminogen activation pathway or its related compound (e.g. plasminogen) of the present invention can be used for preparation of a sustained-release preparation. Exemplary suitable sustained-release preparations include semipermeable matrices of solid hydrophobic polymers having a shape and containing glycoproteins, such as membranes or microcapsules. Exemplary sustained-release matrices include polyesters, hydrogels (e.g. poly(2-hydroxyethyl-methacrylate) (Langer, et al. J. Biomed. Mater. Res., 15: 167-277 (1981); Langer, Chem. Tech., 12: 98-105 (1982)) or polyvinyl alcohol, polylactide (US patent 3773919, EP 58,481), L-glutamic acid and a copolymer of ethyl-L-glutamic acid (Sidman, et al. Biopolymers 22: 547 (1983)), non-degradable ethylene-vinyl acetate (Langer, et al. same as above) or degradable lactic acid-glycolic acid copolymer such as Lupron Depot™ (an injectable microsphere composed of a lactic acid-glycolic acid copolymer and leuprolide acetate), and poly-D-(-)-3-hydroxybutyric acid. □ The compounds, such as ethylene-vinyl acetate and lactic acid-glycolic acid, can sustainably release molecules for more than 100 days, and some hydrogels release proteins for a short time period. Rational strategies for stabilizing proteins can be designed based on the relevant mechanisms. For example, in a case that the mechanism of coacervation is formation of intermolecular S-S bonds through the exchange of thiodisulfide bonds, proteins can be stabilized by modifying sulfhydryl residues, lyophilizing from an acid solution, controlling humidity, using an appropriate additive, and developing a specific polymer matrix composition.

Administration and dosage

The pharmaceutical composition of the present invention can be administered by different methods, such as nasal inhalation, aerosol inhalation, nasal drops or eye drops, an intravenous method, an intraperitoneal method, a subcutaneous method, an intracranial method, an intrathecal method, an intraarterial method (e.g. via the carotid artery), an intramuscular method, and a rectal administration.

Preparations for parenteral administration include sterile aqueous or non-aqueous solutions, suspensions, and emulsions. Non-aqueous solvents include propylene glycol, polyethylene glycol, vegetable oils such as olive oil, and injectable organic esters such as ethyl oleate. Aqueous vectors include water, alcoholic/aqueous solutions, emulsions, and suspensions such as saline and a buffer medium. Parenteral intermedia include a sodium chloride solution, Ringer's dextrose, dextrose, sodium chloride, and fixed oils. Intravenous intermedia include fluids and nutritional supplements, electrolyte supplements, etc. A preservative and other additives, such as an antimicrobial, an antioxidant, a chelator, and inert gas, may be present.

Medical staffs will determine a dosage regimen based on various clinical factors. As well known in the medical field, a dosage regimen for any patient is determined according to a variety of factors, including the body size of a patient, the body surface area, age, a specific compound to be administered, sex, the frequency and path of administration, general health conditions, and other drugs to be administered together. A daily dosage range of the pharmaceutical composition containing plasminogen of the present invention may be, for example, about 0.0001-2000 mg/kg, or about 0.001-500 mg/kg (e.g. 0.02 mg/kg, 0.25 mg/kg, 0.5 mg/kg, 0.75 mg/kg, 10 mg/kg, and 50 mg/kg) of body weight of a patient. For example, a dose may be 1 mg/kg of body weight or 50 mg/kg of body weight, or within a range of 1-50 mg/kg of body weight, or at least 1 mg/kg of body weight. Doses greater or less than these exemplary ranges are also covered, especially in view of the above factors. Intermediate doses within the above ranges also fall within the scope of the present invention. Subjects may be administered with the pharmaceutical composition at such doses daily, every other day, weekly, or according to any other regimen determined by empirical analysis. Exemplary dosage regimens include that the pharmaceutical composition is administered at 0.01-100 mg/kg for consecutive days. It is necessary to assess a therapeutic effect and the safety during administration with the drug of the present invention.

Product or kit

An embodiment of the present invention relates to a product or kit, which includes a component of a plasminogen activation pathway or its related compound (e.g. plasminogen). Preferably, the kit includes a container with a label or package insert. Suitable containers include bottles, vials, syringes, etc. The container can be made from a variety of materials such as glass and plastic. The container contains a composition that can be used to treat the disease or symptoms of the present invention, and has a sterile inlet (e.g. the container may be an intravenous solution pack or vial with a plug that can be penetrated by a hypodermic needle). At least one active ingredient in the composition is a component of a plasminogen activation pathway or its related compound (e.g. plasminogen). The label attached to the container is used to describe that the composition is used to treat the symptoms of the present invention. The product may also include a second container containing a medicinal buffer such as phosphate-buffered saline, a Ringer's solution, and a dextrose solution. The product may also include other substances required from a commercial and user standpoint, which include other buffers, a diluent, a filter, a needle, and a syringe. In addition, the product includes a package insert with instructions for use, which are used to, for example, indicate a user of the composition to administrate the component of the plasminogen activation pathway or its related compound (e.g. plasminogen) and other drugs for treating concomitant diseases to a patient.

EXAMPLES

Plasminogen used in all the following examples was from plasma of a human donator, that is, was isolated from plasma of a human donator and purified by a method optimized with reference to the methods described in the following documents: Kenneth C Robbins, Louis Summaria, David Elwyn, et al. Further Studies on the Purification and Characterization of Human Plasminogen and Plasmin. Journal of Biological Chemistry, 1965, 240 (1): 541-550; Summaria L, Spitz F, Arzadon L, et al. Isolation and characterization of the affinity chromatography forms of human Glu- and Lys-plasminogens and plasmins. J Biol Chem. 1976 Jun 25; 251 (12): 3693-9; HAGAN JJ, ABLONDI FB, DE RENZO EC. Purification and biochemical properties of human plasminogen. J Biol Chem. 1960 Apr; 235: 1005-10. The plasminogen monomer content was greater than 98%.

Example 1 Plasminogen can promote the degradation of amyloid beta-protein (Aβ) in a PBS system

Eppendorf (EP) tubes were divided into ① a blank control group, ② a solvent control group, ③ a plasminogen group, and ④ a plasminogen+tPA group, 4 tubes in each group. 43.3 μL of normal saline, 16 μL of plasminogen solution (0.575 mg/mL), 10 μL of ultra-pure water, and 30.7 μL of PBS (10 mM, pH=7.4, Thermo Fisher, 10010-031) were placed in each tube of the blank control group. 43.3 μL of Aβ40 (1.0 mg/mL, ChinaPeptides, 04010011521), 16 μL of solvent solution (including 10 mM sodium citrate, 2% arginine hydrochloride, and 3% mannitol, pH=7.4), 10 μL of ultra-pure water, and 30.7 μL of PBS were placed in each tube of the solvent control group. 43.3 μL of Aβ40 (1.0 mg/mL), 16 μL of plasminogen solution (0.575 mg/mL), 10 μL of ultra-pure water, and 30.7 μL of PBS were placed in each tube of the plasminogen group. 43.3 μL of Aβ40 (1.0 mg/mL), 8 μL of plasminogen solution (1.15 mg/mL), 8 μL of tPA solution (1.0 mg/mL), 10 μL of lysine solution (0.1 mM), and 30.7 μL of PBS were placed in each tube of the plasminogen+tPA group. Then, the materials in each tube were incubated at 37°C for 3 h, and 100 μL of 0.1% trifluoroacetic acid solution was placed in each tube to terminate the reaction.

A 20% gel was prepared according to instructions of a Tris-Tricine-SDS-PAGE gel preparation kit (Solarbio, P1320). A sample of each group was uniformly mixed with a 4× loading buffer (TaKaRa, e2139) in a volume ratio of 3: 1, the mixture was heated at 100°C for 5 min, cooled, and centrifuged for 1 min, and 20 μL of sample was loaded. Electrophoresis was performed at 30 V for 1 h, and then at 100V to the bottom of the gel. After electrophoresis was completed, the gel was peeled off and stained with a 1‰ Coomassie brilliant blue staining solution (1 g of Coomassie brilliant blue R250 was dissolved in 1000 mL of mixture of ethanol, glacial acetic acid, and purified water in a volume ratio of 5: 2: 13) for 30 min, and destained with a destaining solution (a mixture of purified water, glacial acetic acid, and anhydrous ethanol in a volume ratio of 17: 2: 1) to clear. The gel was quantitatively scanned and photographed by using a gel meter.

The accumulation of amyloid beta-protein (Aβ) is a key factor in the formation of Alzheimer's disease. Aβ40 containing 40 residues and Aβ42 containing 42 residues are deposited in the hippocampus and striatum of the brain to form senile plaques, which are main pathogenic factors of AD[1]. The Aβ40 content and the Aβ42 content in the cerebrospinal fluid have gradually become physiological indicators for clinical diagnosis of Alzheimer's disease.

The results show that the Aβ40 content of the solvent control group does not change, and is defined as 100%; Aβ40 of the plasminogen group is partially degraded in a case where plasminogen is added alone; Aβ40 of the plasminogen+tPA group is degraded obviously in vitro in a case where plasminogen and tPA are added together, and has a significant difference compared to the solvent control group (** indicates P<0.01) (see Fig. 1). It indicates that plasminogen can promote the degradation of Aβ40 in a PBS system.

Example 2 Plasminogen can promote the degradation of amyloid beta-protein (Aβ) in the cerebrospinal fluid of a rabbit

Eppendorf (EP) tubes were divided into ① a blank control group, ② a solvent group, and ③ a plasminogen group, 4 tubes in each group. 43.3 μL of normal saline, 16 μL of plasminogen solution (0.575 mg/mL), and 40.7 μL of cerebrospinal fluid of a rabbit were placed in each tube of the blank control group. 43.3 μL of Aβ40 (1.0 mg/mL, ChinaPeptides, 04010011521), 16 μL of solvent solution (including 10 mM sodium citrate, 2% arginine hydrochloride, and 3% mannitol, pH=7.4), and 40.7 μL of cerebrospinal fluid of a rabbit were placed in each tube of the solvent control group. 43.3 μL of Aβ40 (1.0 mg/mL), 16 μL of plasminogen solution (0.575 mg/mL), and 40.7 μL of cerebrospinal fluid of a rabbit were placed in each tube of the plasminogen group. Then, the materials in each tube were incubated at 37°C for 3 h, and 100 μL of 0.1% trifluoroacetic acid solution was placed in each tube to terminate the reaction.

A 20% gel was prepared according to instructions of a Tris-Tricine-SDS-PAGE gel preparation kit (Solarbio, P1320). A sample of each group was uniformly mixed with a 4× loading buffer (TaKaRa, e2139) in a volume ratio of 3: 1, the mixture was heated at 100°C for 5 min, cooled, and centrifuged for 1 min, and 20 μL of sample was loaded. Electrophoresis was performed at 30 V for 1 h, and then at 100V to the bottom of the gel. After electrophoresis was completed, the gel was peeled off and stained with a 1‰ Coomassie brilliant blue staining solution (1 g of Coomassie brilliant blue R250 was dissolved in 1000 mL of mixture of ethanol, glacial acetic acid, and purified water in a volume ratio of 5: 2: 13) for 30 min, and destained with a destaining solution (a mixture of purified water, glacial acetic acid, and anhydrous ethanol in a volume ratio of 17: 2: 1) to clear. The gel was quantitatively scanned and photographed by using a gel meter.

The results show that the Aβ40 content of the solvent control group does not change and is defined as 100%; and Aβ40 of the plasminogen group is partially degraded in a case where plasminogen is added alone and degraded to 74.81% (see Fig. 2). It indicates that plasminogen can promote the degradation of Aβ40 in the cerebrospinal fluid of the rabbit.

Example 3 Plasminogen promotes the degradation of Aβ40 in cerebral homogenates of a mouse model of Alzheimer's disease and a normal mouse

Four 11-week-old B6SJLTg (APPSwFlLon, PSEN1*M146L*L286V) 6799Vas/Mmjax (FAD) mice (stock number: 034840) (FAD for short) and four C57BL/6 (normal) mice were killed, the whole brain tissue was taken out, weighed, and placed into an Eppendorf (EP) tube, $1\times$ PBS (pH=7.4, Thermo Fisher, 10010-031) was added at a rate of 150 mg of tissue per 1 mL of PBS, the tissue was homogenized at 4°C (1 min/time, 3-4 times), the homogenate was centrifuged at 4°C (at 12000 rpm for 15 min), and a supernatant cerebral homogenate was transferred to a new EP tube.

Eppendorf (EP) tubes were divided into ① a blank control group, ② a solvent control group, and ③ a plasminogen group, and 5 parallels were set for each group. 21.5 μL of normal saline, 4.6 μL of plasminogen solution (2 mg/mL), and 23.9 μL of cerebral homogenate of the mouse were placed in each tube of the blank control group. 21.5 μL of Aβ40 (1.0 mg/mL, ChinaPeptides, 04010011521), 4.6 μL of solvent solution (including 10 mM sodium citrate, 2% arginine hydrochloride, and 3% mannitol, pH=7.4), and 23.9 μL of cerebral homogenate of the mouse were placed in each tube of the solvent control group. 21.5 mL of Aβ40 (1.0 mg/mL), 4.6 μL of plasminogen solution (2 mg/mL), and 23.9 μL of cerebral homogenate of the mouse were placed in each tube of the plasminogen group. Then, the materials in each tube were incubated at 37°C for 6 h, and 50 μL of 0.1% trifluoroacetic acid solution was placed in each tube to terminate the reaction.

A 20% gel was prepared according to instructions of a Tris-Tricine-SDS-PAGE gel preparation kit (Solarbio, P1320). A sample of each group was uniformly mixed with a $4\times$ loading buffer (TaKaRa, e2139) in a volume ratio of 3: 1, the mixture was heated at 100°C for 5 min, cooled, and centrifuged for 1 min, and 20 μL of sample was loaded. Electrophoresis was performed at 30 V for 1 h, and then at 100V to the bottom of the gel. After electrophoresis was completed, the gel was peeled off and stained with a 1‰ Coomassie brilliant blue staining solution (1 g of Coomassie brilliant blue R250 was dissolved in 1000 mL of mixture of ethanol, glacial acetic acid, and purified water in a volume ratio of 5: 2: 13) for 30 min, and destained with a destaining solution (a mixture of purified water, glacial acetic acid, and anhydrous ethanol in a volume ratio of 17: 2: 1) to clear. The gel was photographed and quantitatively scanned by using a biomolecular imager.

The results show that in the cerebral homogenates of the mouse models of Alzheimer's disease, the human amyloid Aβ40 content of the plasminogen group is obviously less than that of the solvent control group, and the difference is extremely significant (*** indicates P<0.001); and in the cerebral homogenates of the normal mice, the amyloid Aβ40 content of the plasminogen group is obviously less than that of the solvent control group, and the difference is extremely significant (P=0.001) (see Fig. 3). It indicates that plasminogen can effectively promote the degradation of human amyloid Aβ40 in the cerebral homogenates of the mouse models of Alzheimer's disease and the normal mice.

Example 4 Plasminogen promotes the degradation of human Aβ40 in cerebral homogenates of mouse models of Alzheimer's disease and normal mice

Four 11-week-old B6SJLTg (APPSwFlLon, PSEN1*M146L*L286V) 6799Vas/Mmjax (FAD) mice (stock number: 034840) (FAD for short) and four C57BL/6 (normal) mice were killed, the whole brain tissue was taken out, weighed, and placed in an Eppendorf (EP) tube, $1\times$ PBS (pH=7.4, Thermo Fisher, 10010-031) was added at a rate of 150 mg of tissue per 1 mL of PBS, the tissue was homogenized at 4°C (1 min/time, 3-4 times), the homogenate was centrifuged at 4°C (at 12000 rpm for 15 min), and a supernatant cerebral homogenate was transferred to a new EP tube for later use.

Eppendorf (EP) tubes were divided into ① a blank control group, ② a solvent control group, and ③ a plasminogen group, and 5 parallels were set for each group. 21.5 μL of normal saline, 4.6 μL of plasminogen solution (2 mg/mL), and 23.9 μL of cerebral homogenate of the mouse were placed in each tube of the blank control group. 21.5 μL of Aβ42 (1.0 mg/mL, ChinaPeptides, 04010011526), 4.6 μL of solvent solution (including 10 mM sodium citrate, 2% arginine hydrochloride, and 3% mannitol, pH=7.4), and 23.9 μL of cerebral homogenate of the mouse were placed in each tube of the solvent control group. 21.5 mL of Aβ42 (1.0 mg/mL), 4.6 μL of plasminogen solution (2 mg/mL), and 23.9 μL of cerebral homogenate of the mouse were placed in each tube of the plasminogen group. Then, the materials in each tube were incubated at 37°C for 6 h, and 50 μL of 0.1% trifluoroacetic acid solution was placed in each tube to terminate the reaction.

A 20% gel was prepared according to instructions of a Tris-Tricine-SDS-PAGE gel preparation kit (Solarbio, P1320). A sample of each group was uniformly mixed with a $4\times$ loading buffer (TaKaRa, e2139) in a volume ratio of 3: 1, the mixture was heated at 100°C for 5 min, cooled, and centrifuged for 1 min, and 20 μL of sample was loaded. Electrophoresis was performed at 30 V for 1 h, and then at 100V to the bottom of the gel. After electrophoresis was completed, the gel was peeled off and stained with a 1‰ Coomassie brilliant blue staining solution (1 g of Coomassie brilliant blue R250 was dissolved in 1000 mL of mixture of ethanol, glacial acetic acid, and purified water in a volume ratio of 5: 2: 13) for 30 min, and destained with a destaining solution (a mixture of purified water, glacial acetic acid, and anhydrous ethanol in a volume ratio of 17: 2: 1) to clear. The gel was photographed and quantitatively scanned by using a biomolecular imager.

The results show that in the cerebral homogenates of the mouse models of Alzheimer's disease, the amyloid Aβ42 content of the plasminogen group is less than that of the solvent control group, and the contents of polymers a, b, and c of the plasminogen group are all less than those of the solvent group, and the difference is extremely significant (* indicates P<0.05, and *** indicates P<0.001); and in the cerebral homogenates of the normal mice, the amyloid Aβ42 content of the plasminogen group is obviously less than that of the solvent control group, the difference is extremely significant (*** indicates P<0.001), the contents of polymers a, b, and c are all less than those of the solvent group, and the difference is extremely significant (*** indicates P<0.001) (see Fig. 4). It indicates that plasminogen can effectively promote the degradation of human Aβ42 and its polymers in the cerebral homogenates of the FAD mice and the normal mice.

Example 5 Plasminogen promotes the recovery of memory function of a mouse model of Alzheimer's disease

B6SJL-Tg (APPSwFILon, PSEN1*M146L*L286V) 6799Vas/Mmjax mice (purchase from Jackson lab, stock number: 034840) (FAD for short) were transgenic model mice commonly used for studying Alzheimer's disease. Twelve 12-week-old female FAD mice were randomly divided into two groups, i.e. a solvent group and an administration group, 6 mice in each group, and six SJLB6 female mice (stock number: 10012) were taken as a normal control group. Plasminogen was injected into each mouse of the administration group via the tail vein at a dose of 1 mg/0.1 mL/day, a solvent solution (including 4% arginine and 2% glycine) was injected into each mouse of the solvent group via the tail vein at the same dose, and no drug was administered to the mice of the normal control group. Administration was performed for 5 consecutive days. The day on which administration was started was denoted as the 1st day, and a Y maze spontaneous alternation test was performed on the 6th day. The Y maze was composed of three identical arms. A food provision apparatus was arranged at the end of each arm, spatial memory ability of an experimental animal can be known by analyzing a food acquisition strategy of the animal, i.e., parameters such as the number of arm entries, time, the number of successes, the number of failures, and routes. The Y maze is usually used for assessing learning and memory function. This test fully exploits the rodent's nature to explore new environments, and the animal must rely on the previous memory to enter a correct arm, which can effectively assess the spatial working memory ability of the animal. In the test, the animal was placed to the end of one arm and allowed to explore freely for a few minutes. After a period of time, the animal was placed in the maze again for formal testing. The order in which the animal entered the arms and the total number of arm entries of the animal were recorded, and when the animal entered different arms in sequence (e.g. 1, 2, 3 and 1, 3, 2), it was recorded as a correct alternation. The animal was placed to the end of one arm, and the order in which the animal entered the arms within 8 min was recorded.

The maximum alternation is equal to the total number of arm entries minus 2, and then the percentage is calculated according to a formula of percentage=actual Alternation/the maximum Alternation × 100%. The finally obtained values include the actual Alternation, the maximum Alternation, the percentage of the two, the total travel distance of the animal, and the total number of arm entries[2].

Alzheimer's disease (AD) is a progressive neurodegenerative disease with insidious onset, which is characterized by cognitive impairment, neurodegeneration, beta-amyloid deposition, neurofibrillary tangles, and neuroinflammation[3]. FAD transgenic mice are commonly used model animals for developing treatment drugs for AD.

Percentage of spontaneous alternation

Percentage of spontaneous alternation=actual Alternation/the maximum Alternation × 100%. The results show that compared with the mouse of the normal control group, the percentage of spontaneous alternation of the mouse of the solvent group is obviously increased; and the percentage of spontaneous alternation of the mouse of administration group is obviously less than that of the mouse of the solvent, the statistical difference is significant (* means P<0.05), and the percentage of spontaneous alternation of the mouse of administration group is closer to that of the mouse of the normal control group (see Fig. 5).

Total number of arm entries

The total number of arm entries refers to the sum of arm entries of a mouse within the prescribed time. The results show that compared with the mouse of the normal control group, the total number of arm entries of the mouse of the solvent group is obviously decreased; the total number of arm entries of the mouse of the administration group is obviously less than that of the mouse of the solvent control group, the statistical difference is significant (* indicates P<0.05), and the total number of arm entries of the mouse of the administration group is closer to that of the mouse of the normal control group (see Fig. 6).

Total travel distance

A total travel distance refers to the total length of motion trails of a mouse within the prescribed time. The results show that compared with the mouse of the normal control group, a total travel distance of the mouse of the solvent group is obviously reduced; a total travel distance of the mouse of the administration group is obviously longer than that of the mouse of the solvent control group, the statistical difference is significant (* indicates P<0.05), and the total travel distance of the mouse of the administration group is closer to that of the mouse of the normal control group (see Fig. 7).

The above results show that plasminogen can promote the recovery spontaneous alternation of the mouse model of Alzheimer's disease, so as to promote memory recovery.

Example 6 Plasminogen reduces the Aβ42 deposition in the cerebral cortex of a mouse model of Alzheimer's disease

Before model construction, twenty 8-week-old male C57 mice were weighed to exclude abnormal mice according to the body weight, and then all the mice were randomly divided into two groups, i.e. a solvent group and an administration group, 10 mice in each group. All the mice were anesthetized, the granule cell layer (positioned according to the coordinates of the front halogen point: AP -2.0 mm, ML ±1.5 mm, DV 2.0 mm) of the hippocampus was positioned according to a stereotaxic map of each mouse. A trace amount (3 μL) of Aβ1-42 oligomer solution was injected into both sides of each mouse of the model group slowly (at an injection velocity of 0.5 μL/min) to construct a model of Alzheimer's disease[3], and a PBS solution was injected into each mouse of a model control group. Preparation of the Aβ1-42 oligomer solution (10 μM): β-Amyloid (1-42) (ChinaPeptides, 04010011521) was added to cold hexafluoroisopropanol to form a solution at a concentration of 1 mg/mL, and the solution was placed at the room temperature for 3 days, subpackaged at a volume of 45 μL/tube, i.e. 10 nmol/mL, placed in a fume hood overnight, dried in a drying oven at 25°C for 1 hour, and preserved at -80°C. When used, 10 μL of dimethyl sulfoxide solution was placed in each tube to redissolve, before injection, 990 μL of sterile PBS was placed in each tube, and the mixture was placed at 4°C for 24 hours and then used. After 21 days of brain stereotactic injection, drugs were administered to the mice of the solvent group and the administration group, and the day on which administration was started was denoted as the 1st day: plasminogen was injected into each mouse of the administration group via the tail vein at a dose of 1 mg/0.1 mL/day, and a solvent solution (including 4% arginine and 2% glycine) was injected into each mouse of the solvent group via the tail vein at a dose of 0.1 mL/day, and administration was performed for 28 consecutive days. On the 29th day, the mice were killed, and the brain tissue was taken out and fixed in 10% formaldehyde for 24-48 h. The fixed brain tissue was dehydrated with graded ethanol, cleared with xylene, and embedded in paraffin. The substantia nigra in a slice with a thickness of 4 μm was positioned, and the slice was subjected to deparaffinage and rehydration, and then washed once with water. The slice was marked by using a PAP pen, incubated in 3% hydrogen peroxide for 15 min, and washed twice with 0.01 M PBS for 5 min each time. The slice was blocked with a 5% normal goat serum (Vector laboratories, Inc., USA) for 30 min; and then, the goat serum was removed, a rabbit anti-mouse Aβ42 antibody (Abcam, ab201060) was added dropwise, and the slice was incubated at 4°C overnight, and washed twice with 0.01 M PBS for 5 min each time. A goat anti-rabbit IgG (HRP) antibody (Abcam) secondary antibody was added, and the slice was incubated at the room temperature for 1 h, and washed twice with PBS for 5 min each time. The slice was developed by using a DAB kit (Vector laboratories, Inc., USA), washed three times with water, and re-stained with hematoxylin for 30 s, and washed with running water for 5 min. The slice was dehydrated with graded ethanol, cleared with xylene, and sealed by a neutral gum. The slice was observed under a 200× optical microscope.

Neurotoxicity of amyloid β-protein (Aβ) plays a major role in the progression of Alzheimer's disease[4].

The results of the above experiment show that the level of Aβ42 deposition in the cerebral cortex of the mouse of the solvent group (see Fig. 8A) is obviously higher than that of the mouse of the administration group (see Fig. 8B), and the statistical difference of the optical density quantitative analysis results is significant (* indicates P<0.05) (see Fig. 8C). It indicates that plasminogen can obviously reduce the Aβ42 deposition in the cerebral cortex of the mouse models of Alzheimer's disease.

Example 7 Plasminogen reduces the Aβ42 level in the brain tissue of a mouse model of Alzheimer's disease

B6SJL-Tg                    (APPSwFlLon, PSEN1*M146L*L286V) 6799Vas/Mmjax mice (purchased from Jackson lab, stock number: 034840) were backcrossed once with C57BL/6J mice to breed offspring (B6-F1-FAD for short). Eighteen 16-17-week-old female B6-F1-FAD mice and nine 9-week-old female C57BL/6J mice were taken. The B6-F1-FAD mice were randomly divided into two groups, i.e. a solvent group and an administration group, according to the body weight and Y maze test results, 9 mice in each group. The nine C57BL/6J mice were taken as a blank control group. After grouping, a solvent solution (including 4% arginine and 2% glycine) was injected into each mouse of the blank control group and the solvent group via the tail vein at a dose of 5 mL/kg. Plasminogen was injected into each mouse of the administration group via the tail vein at a dose of 50 mg/kg for 8 consecutive days. 5 days after drug withdrawal, 7 mice, 7 mice, and 6 mice were respectively randomly selected from the blank group, the solvent group, and the administration group were killed, the brain tissue was taken out and homogenized at 4°C, and a supernate, i.e. a homogenate, was taken for a BCA protein assay for determining the total protein concentration and a Wstem blot assay.

A 16.5% gel was prepared according to instructions of a Tris-Tricine-SDS-PAGE gel preparation kit (Solarbio, P1320). A sample of each group was uniformly mixed with a 4× loading buffer (TaKaRa, e2139) in a volume ratio of 3: 1, the mixture was heated at 100°C for 5 min, cooled, and centrifuged for 2 min, and 100 ug of total protein was loaded. Electrophoresis was performed at 30 V for 1.5 h, and then at 100V to the bottom of the gel. After electrophoresis was completed, the gel was peeled off and transferred to a PVDF membrane (GE, A29433753), and electrophoresis was performed at 15 V for 2.5 h. The transferred PVDF membrane was immersed in a blocking solution (a 5% skim emulsion) and blocked in a refrigerator at 4°C overnight, washed four times with TBST (a 0.01 M Tris-NaCl buffer, pH=7.6), a rabbit anti-mouse Aβ42 antibody (Abcam, ab201060) was added, the PVDF membrane was incubated at the room temperature for 2 h, and washed four times with TBST, a goat anti-rabbit IgG (HRP) antibody (Abcam, ab6721) secondary antibody was added, and the PVDF membrane was incubated at the room temperature for 1 h, washed four times with TBST, placed on a clean imaging plate, developed with Immobilon Western HRP Substrate (MILLIPORE, WBKLS0100), photographed by using a biomolecular imager, and quantitatively analyzed by using Image J.

The results show that a certain level of Aβ42 is present in the cerebral homogenate of the mouse of the blank control group; the Aβ42 level in the brain tissue of the mouse of the solvent group is obviously higher than that in the mouse of the administration group, and the statistical P value is equal to 0.09 (see Fig. 9). It indicates that plasminogen can reduce the Aβ42 level in the brain tissue of the mouse model of Alzheimer's disease.

Example 8 Plasminogen promotes the degradation of Tau proteins in a cerebral homogenate of a normal mouse

Four 11-12-week-old male C57BL/6J mice with a body weight of 18-25 g were killed, the whole brain tissue was taken out and weighed, 1× PBS was (pH=7.4, Thermo Fisher, 10010-031) was added at a rate of 150 mg of tissue per 1 mL of PBS, the tissue was homogenized at 4°C (3-4 times, 1 min/time) and then centrifuged at 4°C (at 12000 rpm for 20 min), and a supernatant, i.e. a homogenate was transferred to a new EP tube.

Eppendorf (EP) tubes were divided into ① a blank group, ② a blank control group, ③ a solvent control group, and ④ a plasminogen group, and 5 parallels were set for each group. 21.5 μL of normal saline, 4.6 μL of solvent solution (including 10 mM, 2% arginine hydrochloride, 3% mannitol, pH=7.4), 23.9 μL of cerebral homogenate of the mouse were placed in each tube of the blank group. 21.5 μL of normal saline, 4.6 μL of plasminogen solution (0.5 mg/mL), and 23.9 μL of cerebral homogenate of the mouse were placed in each tube of the blank control group. 21.5 μL of Tau protein solution (1.0 mg/mL, customized expressed human Tau proteins, GenScript, UniProtKB - P10636-8), 4.6 μL of solvent so-

lution, and 23.9 μL of cerebral homogenate of the mouse were placed in each tube of the solvent control group. 21.5 μL of Tau protein solution (1.0 mg/mL), 4.6 μL of plasminogen solution (0.5 mg/mL), and 23.9 μL of cerebral homogenate of the mouse were placed in each tube of the plasminogen group. Then, the materials in each tube were incubated at 37°C for 6 h, and 50 μL of 0.1% trifluoroacetic acid solution was placed in each tube to terminate the reaction.

A 10% gel was prepared according to instructions of an SDS-PAGE gel preparation kit. A sample of each group was uniformly mixed with a 4× loading buffer (TaKaRa, e2139) in a volume ratio of 3: 1, the mixture was heated at 100°C for 5 min, cooled, and centrifuged for 2 min, and 20 μL of sample was loaded. Electrophoresis was performed at 30 V for 45 min and then at 100 V to the bottom of the gel. After electrophoresis was completed, the gel was peeled off and activated onto a PVDF membrane (GE, A29433753), and electrophoresis was performed at 15 V for 2.5 h. The transferred PVDF membrane was immersed in a blocking solution (a 5% skim emulsion) and blocked in a refrigerator at 4°C overnight, washed four times with TBST (a 0.01 M Tris-NaCl buffer, pH=7.6), a rabbit-derived Tau protein antibody (Abcam, ab151559) was added, the PVDF membrane was incubated at the room temperature for 2 h, and washed four times with TBST, a goat anti-rabbit IgG (HRP) antibody (Abcam, ab6721) secondary antibody was added, and the PVDF membrane was incubated at the room temperature for 1 h, washed four times with TBST, placed on a clean imaging plate, developed with Immobilon Western HRP Substrate (MILLIPORE, WBKLS0100), photographed by using a biomolecular imager, and quantitatively analyzed by using Image J.

Tau proteins are the most abundant microtubule-associated proteins. Tau proteins are phosphate-containing proteins, and a Tau protein molecule in normal mature brain contains 2 or 3 phosphate groups. However, Tau proteins in the brain of a patient with Alzheimer's disease (senile dementia) are abnormally hyperphosphorylated, and each Tau protein molecule may contain 5 to 9 phosphate groups and lose normal biological functions[5].

The results show that in the cerebral homogenates of the normal mice, the Tau protein content of the plasminogen group is obviously less than that of the solvent group, and the difference is significant (* indicates P<005, ** indicates P<0.01, and *** indicates P<0.001) (see Fig. 10). It indicates that plasminogen can promote the degradation of Tau protein in the cerebral homogenate of the normal mouse.

Example 9 Plasminogen promotes the degradation of Tau proteins in a cerebral homogenate of a mouse model of Alzheimer's disease

Four 11-week-old B6SJLTg (APPSwFILon, PSEN1*M146L*L286V) 6799Vas/Mmjax (FAD) mice (stock number: 034840) (FAD for short) were killed, the whole brain was taken out and weighed, and cerebral homogenates were prepared with reference to Example 8 and placed in EP tubes.

Eppendorf (EP) tubes were divided into ① a blank group, ② a blank control group, ③ a solvent control group, and ④ a plasminogen group, and 5 parallels were set for each group. 21.5 μL of normal saline, 4.6 μL of solvent solution (including 10 mM sodium citrate, 2% arginine hydrochloride, and 3% mannitol, pH=7.4), and 23.9 μL of cerebral homogenate of the mouse were placed in each tube of the blank group. 21.5 μL of normal saline, 4.6 μL of plasminogen solution (0.5 mg/mL), and 23.9 μL of cerebral homogenate of the mouse were placed in each tube of the blank control group. 21.5 μL of Tau (1.0 mg/mL, customized expressed human Tau proteins, GenScript, UniProtKB - P10636-8), 4.6 μL of solvent solution, and 23.9 μL of cerebral homogenate of the mouse were placed in each tube of the solvent control group. 21.5 μL of Tau (1.0 mg/mL), 4.6 μL of plasminogen solution (0.5 mg/mL), and 23.9 μL of cerebral homogenate of the mouse were placed in each tube of the plasminogen group. Then, the materials in each tube were incubated at 37°C for 6 h, and 50 μL of 0.1% trifluoroacetic acid solution was placed in each tube to terminate the reaction.

A 10% gel was prepared according to instructions of an SDS-PAGE gel preparation kit. A sample of each group was uniformly mixed with a 4× loading buffer (TaKaRa, e2139) in a volume ratio of 3: 1, the mixture was heated at 100°C for 5 min, cooled, and centrifuged for 2 min, and 20 μL of sample was loaded. Electrophoresis was performed at 30 V for 45 min and then at 100 V to the bottom of the gel. After electrophoresis was completed, the gel was peeled off and activated onto a PVDF membrane (GE, A29433753), and electrophoresis was performed at 15 V for 2.5 h. The transferred PVDF membrane was immersed in a blocking solution (a 5% skim emulsion) and blocked in a refrigerator at 4°C overnight, washed four times with TBST (a 0.01 M Tris-NaCl buffer, pH=7.6), a rabbit-derived Tau protein antibody (Abcam, ab151559) was added, the PVDF membrane was incubated at the room temperature for 2 h, and washed four times with TBST, a goat anti-rabbit IgG (HRP) antibody (Abcam, ab6721) secondary antibody was added, and the PVDF membrane was incubated at the room temperature for 1 h, washed four times with TBST, placed on a clean imaging plate, developed with Immobilon Western HRP Substrate (MILLIPORE, WBKLS0100), photographed by using a biomolecular imager, and quantitatively analyzed by using Image J.

The results show that in the cerebral homogenates of the mouse models of Alzheimer's disease, the Tau protein content of the plasminogen group is obviously less than that of the solvent control group, and the statistical difference is significant (* indicates P<005, ** indicates P<0.01) (see Fig. 11). It indicates that plasminogen can promote the degradation of Tau proteins in the cerebral homogenate of the mouse model of Alzheimer's disease.

Example 10 Plasminogen reduces the Tau protein level in the brain tissue of a mouse model of Alzheimer's disease

B6SJL-Tg (APPSwFlLon, PSEN1*M146L*L286V) 6799Vas/Mmjax mice (purchased from Jackson lab, stock number: 034840) were backcrossed three times with C57BL/6J mice to breed offspring (B6-F3-FAD for short). Eighteen 20-25-week-old female B6-F3-FAD mice and nine 9-week-old female C57BL/6J mice were selected. The B6-F3-FAD mice were randomly divided into two groups, i.e. a solvent group and an administration group, according to the body weight and Y maze test results, 9 mice in each group. The nine C57BL/6J mice were taken as a blank control group. After grouping, a solvent solution (including 4% arginine and 2% glycine) was injected into each mouse of the blank control group and the solvent group via the tail vein at a dose of 5 mL/kg. Plasminogen was injected into each mouse of the administration group via the tail vein at a dose of 50 mg/kg for 28 consecutive days. 7 days after drug withdrawal, mice were randomly selected from each group and killed, the brain tissue was taken out and homogenized at 4°C, and a supernatant, i.e. a cerebral homogenate, was collected and subjected to a BCA protein assay for determining total protein and a Western blot assay.

A 10% gel was prepared according to instructions of an SDS-PAGE gel preparation kit (Solarbio, P1320). A sample of each group was uniformly mixed with a 4× loading buffer (TaKaRa, e2139) in a volume ratio of 3: 1, the mixture was heated at 100°C for 5 min, cooled, and centrifuged for 2 min, and 100 ug of total protein was loaded. Electrophoresis was performed at 30 V for 1.5 h, and then at 100V to the bottom of the gel. After electrophoresis was completed, the gel was peeled off and transferred to a PVDF membrane (GE, A29433753), and electrophoresis was performed at 15 V for 2.5 h. The transferred PVDF membrane was immersed in a blocking solution (a 5% skim emulsion) and blocked in a refrigerator at 4°C overnight, washed four times with TBST (a 0.01 M Tris-NaCl buffer, pH=7.6), a rabbit anti-mouse Tau antibody (Abcam, ab151559) was added, the PVDF membrane was incubated at the room temperature for 2 h, and washed four times with TBST, a goat anti-rabbit IgG (HRP) antibody (Abeam, ab6721) secondary antibody was added, and the PVDF membrane was incubated at the room temperature for 1 h, washed four times with TBST, placed on a clean imaging plate, developed with Immobilon Western HRP Substrate (MILLIPORE, WBKLS0100), photographed by using a biomolecular imager, and quantitatively analyzed by using Image J.

The results show that certain levels of Tau proteins having different molecular weights are present in the cerebral homogenate of the mouse of the blank control group; the levels of Tau proteins having different molecular weights and the level of total protein in the brain tissue of the mouse of the administration group are obviously lower than those in the mouse of the solvent group, and the statistical analysis P values of the two groups in the levels of Tau proteins having molecular weights of 35 kd, 35-40 kd, 40 kd, and 54 kd and the level of total protein are 0.174, 0.0406, 0.052, 0.067, and 0.055, respectively (see Fig. 12). It indicates that plasminogen can promote the degradation of Tau proteins in the brain tissue of the mouse model of Alzheimer's disease.

Example 11 Plasminogen promotes the cleavage of Pro-BDNF in a cerebral homogenate of a mouse model of Alzheimer's disease

Four 11-week-old B6SJLTg (APPSwFlLon, PSEN1*M146L*L286V) 6799Vas/Mmjax (FAD) mice (stock number: 034840) (FAD for short) were killed, the whole brain tissue was taken out, and cerebral homogenates were prepared as described above and transferred in EP tubes.

Eppendorf (EP) tubes were divided into ① a blank group, ② a blank control group, ③ a solvent control group, and ④ an administration group, and 5 parallels were set for each group. 21.5 $\mu$L of normal saline, 4.6 $\mu$L of solvent solution (including 10 mM sodium citrate, 2% arginine hydrochloride, and 3% mannitol, pH=7.4), and 23.9 $\mu$L of cerebral homogenate of the mouse were placed in each tube of the blank group. 21.5 $\mu$L of normal saline, 4.6 $\mu$L of plasminogen solution (2 mg/mL), and 23.9 $\mu$L of cerebral homogenate of the mouse were placed in each tube of the blank control group. 21.5 $\mu$L of Pro-BDNF (1.0 mg/mL, customized expressed, GenScript, UniProtKB - P23560), 4.6 $\mu$L of solvent solution (a citric acid-sodium citrate solution), and 23.9 $\mu$L of cerebral homogenate of the mouse were placed in each tube of the solvent control group. 21.5 mL of Pro-BDNF (1.0 mg/mL), 4.6 $\mu$L of plasminogen solution (2 mg/mL), and 23.9 $\mu$L of cerebral homogenate of the mouse were placed in each tube of the plasminogen group. Then, the materials in each tube were incubated at 37°C for 6 h, and 50 $\mu$L of 0.1% trifluoroacetic acid solution was placed in each tube to terminate the reaction.

A 12% gel was prepared according to instructions of an SDS-PAGE gel preparation kit. A sample of each group was uniformly mixed with a 4× loading buffer (TaKaRa, e2139) in a volume ratio of 3: 1, the mixture was heated at 100°C for 5 min, cooled, and centrifuged for 2 min, and 20 $\mu$L of sample was loaded. Electrophoresis was performed at 30 V for 45min and then at 100 V to the bottom of the gel. After electrophoresis was completed, the gel was peeled off, stained with a 1‰ Coomassie brilliant blue staining solution (1 g of coomassie brilliant blue R250 was dissolved in 1000 mL of mixture of ethanol, acetic acid, and purified water in a volume ratio of 5: 2: 13) for 30 min, and destained with a destaining solution (a mixture of purified water, acetic acid, and anhydrous ethanol in a volume ratio of 17: 2: 1) to clear. The gel was photographed by using a biomolecular imager and subjected to quantitative scanning analysis.

Brain-derived neurotrophic factor (BDNF) is an alkaline protein having a molecular weight of 12.3 kDa, is composed of 119 amino acid residues, and contains three pairs of disulfide bonds. BDNF is present in the body in the form of dimer and synthesized in the form of a BDNF precursor (Pro-BDNF) that can be cleaved by enzymolysis to form mature BDNF. It has been reported in documents that Pro-BDNF has opposite effects to mature BDNF formed by cleaving Pro-BDNF. Pro-BDNF promotes apoptosis of nerve cells and reduces neural synaptic plasticity[6]. Mature BDNF and its receptors are widely found in the central nervous system, and play an important role in in the survival, differentiation, and growth and development of neurons during the development of the central nervous system. Furthermore, they can prevent neuronal damage and apoptosis, improve the pathological state of neurons, promote biological effects, such as regeneration and differentiation, of injured neurons, and are also necessary for the survival and normal physiological functions of neurons in the mature central and peripheral nervous systems[7].

The results show that in the cerebral homogenates of mouse models of Alzheimer's disease, the Pro-BDNF content of the plasminogen group is obviously less than that of the solvent group, and the difference is extremely significant (* indicates P<0.05, and *** indicates P<0.001) (see Fig. 13). It indicates that plasminogen can promote the cleavage of Pro-BDNF in the cerebral homogenate of the mouse model of Alzheimer's disease.

Example 12 Plasminogen promotes the cleavage of Pro-BDNF in a cerebral homogenate of a mouse model of Alzheimer's disease to form mature BDNF

Four 11-week-old B6SJLTg (APPSwFlLon, PSEN1*M146L*L286V) 6799Vas/Mmjax (FAD) mice (stock number: 034840) (FAD for short) were killed, the whole brain tissue was taken out, and cerebral homogenates were prepared as described above and transferred in EP tubes.

Eppendorf (EP) tubes were divided into ① a blank group, ② a blank control group, ③ a solvent control group, and ④ an administration group, and 5 parallels were set for each group. 21.5 $\mu$L of normal saline, 4.6 $\mu$L of solvent solution (including 10 mM sodium citrate, 2% arginine hydrochloride, and 3% mannitol, pH=7.4), and 23.9 $\mu$L of cerebral homogenate of the mouse were placed in each tube of the blank group. 21.5 $\mu$L of normal saline, 4.6 $\mu$L of plasminogen solution (2 mg/mL), and 23.9 $\mu$L of cerebral homogenate of the mouse were placed in each tube of the blank control group. 21.5 $\mu$L of Pro-BDNF (1.0 mg/mL, customized expressed, GenScript, UniProtKB - P23560), 4.6 $\mu$L of solvent solution

(a citric acid-sodium citrate solution), and 23.9 μL of cerebral homogenate of the mouse were placed in each tube of the solvent control group. 21.5 mL of Pro-BDNF (1.0 mg/mL), 4.6 μL of plasminogen solution (2 mg/mL), and 23.9 μL of cerebral homogenate of the mouse were placed in each tube of the plasminogen group. Then, the materials in each tube were incubated at 37°C for 6 h, and 50 μL of 0.1% trifluoroacetic acid solution was placed in each tube to terminate the reaction.

A 12% gel was prepared according to instructions of an SDS-PAGE gel preparation kit. A sample of each group was uniformly mixed with a 4× loading buffer (TaKaRa, e2139) in a volume ratio of 3: 1, the mixture was heated at 100°C for 5 min, cooled, and centrifuged for 2 min, and 20 μL of sample was loaded. Electrophoresis was performed at 30 V for 45min and then at 100 V to the bottom of the gel. After electrophoresis was completed, the gel was peeled off and transferred to a PVDF membrane (GE, A29433753), and electrophoresis was performed at 15 V for 2.5 h. The transferred PVDF membrane was immersed in a blocking solution (a 5% skim emulsion) and blocked in a refrigerator at 4°C overnight, washed four times with TBST (a 0.01 M Tris-NaCl buffer, pH=7.6), a rabbit anti-human BDNF antibody (Boster Biological Technology, PB9075) was added, the PVDF membrane was incubated at the room temperature for 3 h, and washed four times with TBST, a goat anti-rabbit IgG (HRP) antibody (Abcam, ab6721) secondary antibody was added, the PVDF membrane was incubated at the room temperature for 1 h, washed four times with TBST, placed on a clean imaging plate, developed with Immobilon Western HRP Substrate (MILLIPORE, WBKLS0100), and photographed by using a biomolecular imager, and optical densities of bands were quantitatively analyzed by using Image J.

The results show that in the cerebral homogenates of the mouse models of Alzheimer's disease, the Pro-BDNF content of the plasminogen group is obviously less than that of the solvent control group, and the difference is extremely significant (** indicates P<0.01, and ** indicates P<0.001); and the BDNF content of the plasminogen group is obviously higher than that of the solvent control group, and the difference is extremely significant (see Fig. 14). It indicates that plasminogen can promote the cleavage of Pro-BDNF and formation of mature BDNF in the cerebral homogenate of the mouse model of Alzheimer's disease.

Example 13 Plasminogen promotes the expression of BDNF in the hippocampus of a mouse model of Alzheimer's disease

Before model construction, twenty-three 24-week-old male C57 mice were weighed, abnormal mice were excluded according to the body weight, and then all the mice were randomly divided into two groups, i.e. a blank control group and a model group, 7 mice in the blank control group and 16 mice in the model group. All the mice were anesthetized, and models of Alzheimer's disease were constructed with reference to Example 6. After 28 days of brain stereotaxic injection, all the mice were weighed and tested by a Y maze, abnormal mice of the blank control group and the model group were excluded according to the test results. The mice of the model group were randomly divided into two groups, i.e. a solvent group and an administration group, 6 mice in the solvent group, 7 mice in the administration group, and 6 mice in the blank control group. Drugs were administered to the mice of the solvent group and the administration group, and the day on which administration was started was denoted as the 1st day. Plasminogen was injected into each mouse of the administration group via the tail vein at a dose of 1 mg/0.1 mL/day, a solvent solution (including 4% arginine and 2% glycine) was injected into each mouse of the solvent group via the tail vein at a dose of 0.1 mL/day, administration was performed for 28 consecutive days, and no drug was administered to the mice of the blank control group. On the 29th day, the mice were killed, and the brain tissue was taken out and fixed in 10% formaldehyde for 24-48 h. The fixed brain tissue was dehydrated with graded ethanol, cleared with xylene, and embedded in paraffin. The substantia nigra in a slice with a thickness of 4 μm was positioned, and the slice was subjected to deparaffinage and rehydration, and then washed once with water. The slice was marked by using a PAP pen, incubated in 3% hydrogen peroxide for 15 min, and washed twice with 0.01 M PBS for 5 min each time. The slice was blocked with a 5% normal goat serum (Vector laboratories, Inc., USA) for 30 min; and then, the goat serum was removed, a rabbit anti-mouse BDNF antibody (BosterBio, PB9075) was added dropwise, and the slice was incubated at 4°C overnight, and washed twice with 0.01 M PBS for 5 min each time. A goat anti-rabbit IgG (HRP) antibody (Abcam) secondary antibody was added, and the slice was incubated at the room temperature for 1 h, and washed twice with PBS for 5 min each time. The slice was developed by using a DAB kit (Vector laboratories, Inc., USA), washed three times with water, and re-stained with hematoxylin for 30 s, and washed with running water for 5 min. The slice was dehydrated with graded ethanol, cleared with xylene, and sealed by a neutral gum. The slice was observed under a 200× optical microscope.

The results show that a certain level of BDNF (indicated by arrows) is expressed in the hippocampus of the mouse of the blank control group (see Fig. 15A); the expression of BDNF in the hippocampus of the mouse of the solvent group (see Fig. 15B) is obviously lower than that of the mouse of the blank control group; the expression of BDNF in the hippocampus of the mouse of the administration group (see Fig. 15C) is obviously higher than that of the mouse of the solvent group, and the statistical difference is significant (* indicates P<0.05) (see Fig. 15D). It indicates that plasminogen can promote the expression of BDNF in the hippocampus of the mouse model of Alzheimer's disease.

Example 14 Plasminogen promotes the cleavage of Pro-NGF in a cerebral homogenate of a mouse model of Alzheimer's disease to form mature NGF

Four 11-week-old B6SJLTg (APPSwFlLon, PSEN1*M146L*L286V) 6799Vas/Mmjax (FAD) mice (stock number: 034840) (FAD for short) were killed, the whole brain tissue was taken out, and cerebral homogenates were prepared in EP tubes as described above.

Eppendorf (EP) tubes were divided into ① a blank control group, ② a blank group, ⑤ a solvent control group, and ④ a plasminogen group, and 5 parallels were set for each group. 21.5 μL of normal saline, 4.6 μL of solvent solution (including 10 mM sodium citrate, 2% arginine hydrochloride, and 3% mannitol, pH=7.4), and 23.9 μL of cerebral homogenate of the mouse were placed in each tube of the blank control group. 21.5 μL of normal saline, 4.6 μL of plasminogen solution (2 mg/mL), and 23.9 μL of cerebral homogenate of the mouse were placed in each tube of the blank group. 21.5 μL of Pro-NGF solution (1.0 mg/mL, customized expressed human Pro-NGF, GenScript, sequence source: UniProtKB - P01138), 4.6 μL of solvent solution, and 23.9 μL of cerebral homogenate of the mouse were placed in each tube of the solvent control group. 21.5 μL of Pro-NGF solution (1.0 mg/mL), 4.6 μL of plasminogen solution (2 mg/mL), and 23.9 μL of cerebral homogenate of the mouse were placed in each tube of the plasminogen group. Then, the materials in each tube were incubated at 37°C for 6 h, and 50 μL of 0.1% trifluoroacetic acid solution was placed in each tube to terminate the reaction.

A 15% gel was prepared according to instructions of an SDS-PAGE gel preparation kit. A sample of each group was uniformly mixed with a 4× loading buffer (TaKaRa, e2139) in a volume ratio of 3: 1, the mixture was heated at 100°C for 5 min, cooled, and centrifuged for 2 min, and 20 μL of sample was loaded. Electrophoresis was performed at 30 V for 30min and then at 100 V to the bottom of the gel. After electrophoresis was completed, the gel was peeled off and activated onto a PVDF membrane (GE, A29433753), and electrophoresis was performed at 15 V for 2.5 h. The transferred PVDF membrane was immersed in a blocking solution (a 5% skim emulsion) and blocked in a refrigerator at 4°C overnight, washed four times with TBST (a 0.01 M Tris-NaCl buffer, pH=7.6), a rabbit anti-human NGF antibody (Abcam, ab52918) was added, the PVDF membrane was incubated at the room temperature for 2 h, and washed four times with TBST, a goat anti-rabbit IgG (HRP) antibody (Abcam, ab6721) secondary antibody was added, the PVDF membrane was incubated at the room temperature for 1 h, washed four times with TBST, placed on a clean imaging plate, developed with Immobilon Western HRP Substrate (MILLIPORE, WBKLS0100), and photographed by using a biomolecular imager, and optical densities of bands were quantitatively analyzed by using Image J.

Nerve growth factor (NGF) is an important member of the neurotrophic factor family. It is synthesized in vivo in the form of precursor, and includes signal peptide, leader peptide, and mature peptide. Researches have reported that the nerve growth factor (NGF) precursor (Pro-NGF) has opposite effects to NGF formed by cleaving Pro-NGF. Pro-NGF can promote apoptosis of nerve cells. Mature NGF participates in the regulation of growth, development, differentiation, survival, post-injury repair, and other processes of nerve cells, and also plays an important role in regulating the functional expression of central and peripheral neurons[8].

The results show that in the cerebral homogenates of the mouse models of Alzheimer's disease, the Pro-NGF content of the plasminogen group is obviously less than that of the solvent control group, and the difference is extremely significant (*** indicates P<0.001); the NGF content of the plasminogen group is obviously greater than that of the solvent control group, and the difference is significant (see Fig. 16). It indicates that plasminogen can promote the cleavage of Pro-NGF and formation of mature NGF in the cerebral homogenate of the mouse model of Alzheimer's disease.

Example 15 Plasminogen promotes the recovery of anxiety and depression behaviors of a mouse model of Alzheimer's disease

Before model construction, 28 male C57 mice were weighed, abnormal mice were excluded according to the body weight, and then all the mice were randomly divided into two groups, i.e. a blank control group and a model group, 8 mice in the blank control group and 20 mice in the model group. After grouping, models of Alzheimer's disease were constructed with reference to Example 6[3]. After 65 days of brain stereotaxic injection, all the mice were tested by a water maze, and abnormal mice of a model control group (i.e. the blank control group) and the model group were excluded according to the test results. The mice of the model group were randomly divided into two groups, i.e. a solvent group and an administration group, 10 mice in the solvent group, 10 mice in the administration group, and 8 mice in the blank control group. After grouping, the first stage of administration was performed on the mice of the solvent group and the administration group: plasminogen was injected into each mouse of the administration group via the tail vein at a dose of 50 mg/kg/day, a solvent solution (including 4% arginine and 2% glycine) was injected to each mouse of the solvent group and the blank control group via the tail vein at a dose of 5 mL/kg/day, administration was performed for 28 consecutive days. 50 days after the first stage of administration was completed, the second stage of administration was performed in the same way as the first stage for 7 consecutive days. An open field test was performed on the 8th day of the second stage of administration.

Open field test

In the test, the mouse was placed in the central of the bottom of the open field (40×40×40 cm) while video recording and timing were performed at the same time. The mouse was continuously observed for 5 min, and 3 tests were performed on each mouse. The Smart system is a complete and user-friendly video tracking system for assessing behaviors of an experimental animal. It records trajectories, activities, specific behaviors (e.g. rotation, stretching, and feeding), and events, and calculates various analysis parameter. The test used the Smart3.0 system to record and analyze motions of the mice, and parameters included a total boundary zone travel distance and a central zone travel distance. In each test, the box was wiped with 70% ethanol to prevent the preference caused by odor[1].

The open field test is designed based on the phobotaxis of mice, which means that mice are afraid of open, unknown, and potentially dangerous places, and thus have a natural tendency to move "against the wall". A total distance and an average speed are regarded as main data reflecting spontaneous activities of a mouse, the phobotaxis is assessed based on activities of the mouse in surrounding zones (four corners and four sides) of the open field. In view of duration in the surrounding zones that reflects the phobotaxis, if the duration is shorter, the mouse is more "adventurous". If the duration in the central zone is longer, the phobotaxis and the anxiety (depression) level are lower.

Percentage of boundary zone travel distance

The percentage of boundary zone travel distance refers to a ratio of the length of motion trails of a mouse in a boundary zone to the total length of motion trails within the specified time The results show that the mouse of the blank control group has certain percentage of boundary zone travel distance; the percentage of boundary zone travel distance of the mouse of the solvent group is obviously greater than that of the mouse of the blank control group; the percentage of boundary zone travel distance of the mouse of the administration group is obviously less than that of the mouse of the solvent group, and the statistical difference is close to significant (P=0.08) (see Fig. 17). It indicates that plasminogen can promote the recovery of anxiety and depression behaviors of the mouse model of Alzheimer's disease.

Percentage of central zone travel distance

The percentage of central zone travel distance refers to a ratio of the length of motion trails of a mouse in the central zone to the total length of motion trails within the specified time. The results show that the mouse of the blank control group has certain percentage of central zone travel distance; the percentage of central zone travel distance of the mouse of the solvent group is obviously less than that of the mouse of the blank control group; the percentage of central zone travel distance of the mouse of the administration is obviously greater than that of the mouse of the solvent group, and the statistical difference is close to significant (P=0.08) (see Fig. 18). It indicates that plasminogen can promote the recovery of anxiety and depression behaviors of the mouse model of Alzheimer's disease.

Example 16 Plasminogen promotes the recovery of anxiety and depression behaviors of a mouse model of Alzheimer's disease

Before model construction, 28 male C57 mice were weighed, abnormal mice were excluded according to the body weight, and then all the mice were randomly divided into two groups, i.e. a blank control group and a model group, 8 mice in the blank control group and 20 mice in the model group. After grouping, models of Alzheimer's disease were constructed with reference to Example 6[3]. After 65 days of brain stereotaxic injection, all the mice were tested by a water maze, and abnormal mice of a model control group (i.e. the blank control group) and the model group were excluded according to the test results. The mice of the model group were randomly divided into two groups, i.e. a solvent group and an administration group, 10 mice in the solvent group, 10 mice in the administration group, and 8 mice in the blank control group. After grouping, the first stage of administration was performed on the mice of the solvent group and the administration group: plasminogen was injected into each mouse of the administration group via the tail vein at a dose of 50 mg/kg/day, a solvent solution (including 4% arginine and 2% glycine) was injected to each mouse of the solvent group and the blank control group via the tail vein at a dose of 5 mL/kg/day, administration was performed for 28 consecutive days. 50 days after the first stage of administration was completed, the second stage of administration was performed in the same way as the first stage for 9 consecutive days. An elevated plus maze behavioral test was performed two days after the second stage of administration was completed.

The elevated plus maze test is used to assess the anxiety state of an animal based on conflicting behaviors formed by the exploratory nature of animals for new and different environments and the fear of high hanging open arms. The elevated plus maze has a pair of open arms and a pair of closed arms. Rodents tend to move in the closed arms due to their dark addiction, but they also move in the open arms out of curiosity and exploration. In the face of novel stimuli, animals have the impulse to explore and fear at the same time to form conflicting behaviors of exploration and avoidance, resulting in anxiety. However, anti-anxiety drugs can obviously increase the number of open arm entries and the duration. The plus maze is higher than the ground, which is equivalent to that a person stands on a cliff, so that an experimental subject develops fear and anxiety. The elevated plus maze test is widely used in the fields of scientific-research and computer-aided teaching in multiple disciplines such as new drug development/screening/assessment, pharmacology, toxicology, preventive medicine, neurobiology, animal psychology, and behavioral biology, and is a classical experiment in behavioral research, especially anxiety and depression research carried out by medical schools and scientific research institutions.

At the beginning of the test, the mouse was placed in the central grid of the maze and faced to the closed arm, and its activities within 5 minutes were recorded. Observation indicators included: the number of open arm entries (the two forepaws must entry the arm), open arm duration, the number of closed arm entries, and closed arm duration. The percentage of open arm duration, the percentage of the number of open arm entries, and the total number of elevated plus maze entries were calculated. After the test was completed, the mouse was taken out, the two arms were cleaned, and ethanol was sprayed to remove odor. Finally, data was analyzed by using animal behavior software.

Total travel distance

A total travel distance refers to the total length of motion trails of a mouse within the specified recording time. The results show that the mouse of the blank control group has a certain total travel distance; a total travel distance of the mouse of the solvent group is obviously longer than that of the mouse of the blank control group; a total travel distance of the mouse of the administration group is obviously shorter than that of the mouse of the solvent group, the statistical difference is extremely significant (* indicates $P<0.05$, and ** indicates $P<0.01$) (see Fig. 19), and the total travel distance of the mouse of the administration group is closer to that of the mouse of the blank control group. It indicates that plasminogen can promote the recovery of anxiety and depression behaviors of the mouse model of Alzheimer's disease.

Closed arm travel distance

A closed arm travel distance refers to the length of motion trails in a closed arm within the specified time. The results show that the mouse of the blank control group has a certain closed arm travel distance; a closed arm travel distance of the mouse of the solvent group is obviously longer than that of the mouse of the blank control group; a closed arm travel distance of the mouse of the administration group is obviously shorter than that of the mouse of the solvent group, the statistical difference between the two groups is significant (* indicates $P<0.05$, and ** indicates $P<0.01$) (see Fig. 20), the closed arm travel distance of the mouse of the administration group is closer to that of the mouse of the blank control group. It indicates that plasminogen can promote the recovery of anxiety and depression behaviors of the mouse model of Alzheimer's disease.

Percentage of closed arm travel distance

The percentage of closed arm travel distance refers to a ratio of the length of motion trails in a closed arm to the total length of motion trails within the specified time. The results show that the mouse of the blank control group has certain percentage of closed arm travel distance; the percentage of closed arm travel distance of the mouse of the solvent group is obviously greater than that of the mouse of the blank control group; the percentage of closed arm travel distance of the mouse of the administration group is obviously less than that of the mouse of the solvent group, the statistical difference between the two groups is significant (* indicates $P<0.05$) (see Fig. 21), the percentage of closed arm travel distance of the mouse of the administration group is closer to that of the mouse of the blank control group. It indicates that plasminogen can promote the recovery of anxiety and depression behaviors of the mouse model of Alzheimer's disease.

The number of closed arm entries

The results show that the mouse of the blank control group has certain number of closed arm entries; the number of closed arm entries of the mouse of the solvent group is obviously greater than that of the mouse of the blank control group; the number of closed arm entries of the mouse of the administration group is obviously less than that of the mouse of the solvent group, the statistical difference between the two groups is extremely significant (* indicates $P<0.05$, and ** indicates $P<0.01$) (see Fig. 22), the number of closed arm entries of the mouse of the administration group is closer to that of the mouse of the blank control group. It indicates that plasminogen can promote the recovery of anxiety and depression behaviors of the mouse model of Alzheimer's disease.

Closed arm duration

Closed arm duration refers to duration of a mouse in a closed arm within the specified time. The results show that the mouse of the blank control group has certain closed arm duration; closed arm duration of the mouse of the solvent group is obviously shorter than that of the mouse of the blank control group; closed arm duration of the mouse of the administration group is obviously longer than that of the mouse of the solvent group, the statistical difference between the two groups is significant (* indicates $P<0.05$, and ** indicates $P<0.01$) (see Fig. 23), and the closed arm duration of the mouse of the administration group is closer to that of the mouse of the blank control group. It indicates that plasminogen can promote the recovery of anxiety and depression behaviors of the mouse model of Alzheimer's disease.

Percentage of closed arm duration

The percentage of closed arm duration refers to a ratio of time spent by a mouse in a closed arm to total recording time. The results show that the mouse of the blank control group has certain percentage of closed arm duration; the percentage of closed arm duration of the mouse of the solvent group is obviously less than that of the mouse of the blank control group; the percentage of closed arm duration of the mouse of the administration group is obviously greater than that of the mouse of the solvent group, the statistical difference between the two groups is significant (* indicates P<0.05, and ** indicates P<0.01) (see Fig. 24), and the percentage of closed arm duration of the mouse of the administration group is closer to that of the mouse of the blank control group. It indicates that plasminogen can promote the recovery of anxiety and depression behaviors of the mouse model of Alzheimer's disease.

Closed arm average speed

A closed arm average speed refers to a ratio of a closed arm travel distance to closed arm duration. The results show that the mouse of the blank control group has a certain closed arm average speed; a closed arm average speed of the mouse of the solvent group is higher than that of the mouse of the blank control group; a closed arm average speed of the mouse of the administration group is obviously lower than that of the mouse of the solvent group, the statistical difference between the two groups is significant (** indicates P<0.01) (see Fig. 25), and the closed arm average speed of the mouse of the administration group is closer to that of the mouse of the blank control group. It indicates that plasminogen can promote the recovery of anxiety and depression behaviors of the mouse model of Alzheimer's disease.

Example 17 Plasminogen promotes the recovery of memory function of a mouse model of Alzheimer's disease

Before model construction, 28 male C57 mice were weighed, abnormal mice were excluded according to the body weight, and then all the mice were randomly divided into two groups, i.e. a blank control group and a model group, 8 mice in the blank control group and 20 mice in the model group. After grouping, models of Alzheimer's disease were constructed with reference to Example 6[3]. After 65 days of brain stereotaxic injection, all the mice were tested by a water maze, and abnormal mice of a model control group (i.e. the blank control group) and the model group were excluded according to the test results. The mice of the model group were randomly divided into two groups, i.e. a solvent group and an administration group, 10 mice in the solvent group, 10 mice in the administration group, and 8 mice in the blank control group. After grouping, the first stage of administration was performed on the mice of the solvent group and the administration group: plasminogen was injected into each mouse of the administration group via the tail vein at a dose of 50 mg/kg/day, a solvent solution (including 4% arginine and 2% glycine) was injected to each mouse of the solvent group and the blank control group via the tail vein at a dose of 5 mL/kg/day, administration was performed for 28 consecutive days. 50 days after the first stage of administration was completed, the second stage of administration was performed in the same way as the first stage for 9 consecutive days. A Y maze behavioral test was performed two days after the second stage of administration was completed.

The results show that compared with the mouse of the blank control group, the percentage of spontaneous alternation of the mouse of the solvent group is obviously decreased; and the percentage of spontaneous alternation of the mouse of the administration group is obviously greater than that of the mouse of the solvent group, the statistical difference between the two groups is significant (* indicates P<0.05), the percentage of spontaneous alternation of the mouse of the administration group is closer to that in the mouse of the blank control group (see Fig. 26). It indicates that plasminogen can promote the recovery of memory function of the mouse model of Alzheimer's disease.

Example 18 Plasminogen promotes the recovery of anxiety and depression behaviors of a mouse model of Alzheimer's disease

Eighteen 20-25-week-old female JL-Tg (APPSwFlLon, PSEN1*M146L*L286V) 6799Vas/Mmjax mice (parent mice were purchased from Jackson lab, stock number: 034840) were randomly divided into two groups, i.e. a solvent group and an administration group, according to the body weight and Y maze test results, 9 mice in each group. Nine 20-25-week-old C57 female mice were taken as a blank control group. After grouping, a solvent solution (including 4% arginine and 2% glycine) was injected into each mouse of the blank control group and the solvent group via the tail vein at a dose of 5 mL/kg. Plasminogen was injected into each mouse of the administration group via the tail vein at a dose of 50 mg/kg for 18 consecutive days. An elevated plus maze behavioral test was performed on the 19th day.

The results show that the mouse of the blank control group has a certain closed arm travel distance; a closed arm travel distance of the mouse of the solvent group is obviously shorter than that of the mouse of the blank control group; and a closed arm travel distance of the mouse of the administration group is obviously longer than that of the mouse of the solvent group, the statistical difference between the two groups is significant (** indicates P<0.01, and *** indicates P<0.001) (see Fig. 27), and the closed arm travel distance of the mouse of the administration group is closer to that of the mouse of the blank control group. It indicates that plasminogen can promote the recovery of anxiety and depression behaviors of the mouse model of Alzheimer's disease.

Example 19 Plasminogen improves hippocampal damage in a mouse model of Alzheimer's disease

B6SJL-Tg (APPSwFILon, PSEN1*M146L*L286V) 6799Vas/Mmjax mice (purchased from Jackson lab, stock number: 034840) were backcrossed once with C57BL/6J mice to breed offspring (B6-F1-FAD for short). Eighteen 16-17-week-old female B6-F1-FAD mice and nine 9-week-old female C57BL/6J mice were taken. The B6-F1-FAD mice were randomly divided into two groups, i.e. a solvent group and an administration group, according to the body weight and Y maze test results, 9 mice in each group. The nine C57BL/6J mice were taken as a blank control group. After grouping, a solvent solution (including 4% arginine and 2% glycine) was injected into each mouse of the blank control group and the solvent group via the tail vein at a dose of 5 mL/kg. Plasminogen was injected into each mouse of the administration group via the tail vein at a dose of 50 mg/kg for 8 consecutive days. 5 days after drug withdrawal, the mice were killed, and the brain tissue was taken out and fixed in a 10% neutral formaldehyde solution for 24-48 h. The fixed brain tissue was dehydrated with graded ethanol, cleared with xylene, and embedded in paraffin. A slice with a thickness of 3 μm was taken, subjected to deparaffinage and rehydration, and stained with hematoxylin and eosin (HE staining). The stained slice was differentiated with 1% hydrochloric acid alcohol, returned to blue with ammonia water, dehydrated with graded ethanol, and sealed. The slice was placed under a 200× optical microscope, and hippocampus was observed.

The results show that the morphology of the hippocampus of the mouse of the blank control group (see Fig. 28A) is normal; and compared with the mouse of the solvent group (see Fig. 28B), the morphology of the hippocampus of the mouse of the administration group (see Fig. 28C) is obviously improved. It indicates that plasminogen can improve hippocampal damage in the mouse model of Alzheimer's disease.

Example 20 Therapeutic effects on patients with Alzheimer's disease who voluntarily received plasminogen therapy

All the following patients signed informed consent, voluntarily used the drug, and were approved by the hospital ethics committee.

Patient 1, male, 76-year-old, developed memory loss half a year ago, became stubborn, had declined memory ability and ability to learn new things, and was clinically diagnosed with Alzheimer's disease. Dosage regimen: 50 mg of drug was administered by intravenous injection on the first day, the dose was increased at a rate of 10 mg/day from the second day, and meanwhile, 10 mg of the drug was administered by aerosol inhalation from the third day. Administration was performed once a day for 13 consecutive days.

Therapeutic effect: family members stated that during administration, the patient's mental state was gradually improved, the response sensitivity was gradually and significantly improved, and the patient's ability to learn new things and memory were improved in the later period of administration. After 13 days of administration, the general bodily sensations are improved about 50%, and the memory is improved about 50%.

It indicates that plasminogen can improve memory, learning ability, and mental state of the patient with Alzheimer's disease.

Patient 2, female, 96-year-old. Medical history: the patient had been suffering from hypertension for more than 20 years and did not have heart disease and diabetes. The patient developed memory loss about half a year ago, had declined learning ability, thinking ability, and communication ability, was irritable and indifferent, and got 10 points in mini-mental state examination (MMSE).

Dosage regimen: the drug was administered by aerosol inhalation combined with intravenous injection. On the first day, 5 mg of drug was administered by aerosol inhalation three times. From the second day, the same dose of drug was administered by aerosol inhalation at the same frequency as the first day, meanwhile, 30 mg of drug was administered by intravenous injection, and the dose was increased at a rate of 10 mg/day from the third day. Administration was performed for 7 consecutive days.

The mini-mental state examination (MMSE) can comprehensively, accurately, and rapidly reflect the mental state and the degree of cognitive impairment of subjects. MMSE is easy to operate and widely applied at home and abroad, and is the first choice for screening dementia. Score reference: points from 27 to 30 refer to normal; points less than 27 refer to cognitive impairment; points from 21 to 26 refer to mild; points from 10 to 20 refers to moderate; and points from 0 to 9 refer to severe.

Therapeutic effect: 1. the mental state was improved; 2. the symptoms of senile dementia of the patient were improved, and the memory was improved; 3. the anxiety of the patient was reduced; 4. got 10 points in MMSE, mainly reflected in the ability to act with other people's instructions. MMSE scores before and after administration are shown in Table 1.

It indicates that plasminogen can increase MMSE scores of the patient with Alzheimer's disease, and improve the memory, thinking ability, anxiety, and the mental state of the patient.

Table 1 MMSE scores before and after administration

|  | MMSE score |
|---|---|
| Before administration | 10 |
| After administration | 14 |

Patient 3, female, 87-year-old, had poor memory and comprehension ability, was diagnosed with cerebral infarction by a doctor after inpatient examination, with affected memory and comprehension ability, showed the phenomenon of senile dementia, mild confusion, and subdelirium, could not correctly identify the surrounding environment, had confusion of time and place, and impaired communication with people, and got 10 points in the general condition assessment. (For the general condition assessment, the condition of the patient on the first day without receiving the drug was considered as 10, from the second day, i.e. the day on which administration was started, 10 was considered as the worst, 1 was considered as the mildest, and 0 was consider as normal).

Dosage regimen: 150-250 mg of drug was administered by intravenous injection, and meanwhile, 10 mg of drug was administered by aerosol inhalation three times a day, i.e. every 4 hours. Administration was performed for 14 days. 1 week after drug withdrawal, administration was performed for 1 week in the same way. 15 mg of drug was administered by aerosol inhalation three times a day, i.e. every 4 hours. 1 week after drug withdrawal, administration was performed every other day for 2 weeks. 250 mg of drug was administered by intravenous injection, and meanwhile, 15 mg of drug was administered by aerosol inhalation three times a day, i.e. every 4 hours. Then, administration was performed twice a week for 2 weeks. 400 mg of drug was administered by intravenous injection, and meanwhile, 10 mg of drug was administered by aerosol inhalation twice a day. Finally, 500 mg of drug was administered by intravenous injection once a week. Meanwhile, 10 mg of drug was administered by aerosol inhalation twice, and administration was performed twice a week.

During treatment, the above symptoms were gradually improved. After 14 days of administration, the patient's mood turned better, and communication with others was basically unimpeded, memory was restored, the concept of time gradually became clearer, and the patient got 4 points in the general condition assessment. In 70%-80% of the cases where the patients communicated with others, the patient could understand and answer accurately. Although the patient often made mistakes in people's names, but she had no problem with the orientation of a specific person.

It indicates that plasminogen can improve Alzheimer's disease, for example, improves memory, communication skills, cognitive ability, and orientation ability of the patient.

Patient 4, female, 91-year-old, was diagnosed with mild cerebral wilt and mild cognitive impairment. Before administration, the patient got 10 points in the memory assessment, 10 points in the computing power assessment, and 10 points in the orientation assessment (for the general condition assessment, the condition of the patient on the first day without receiving the drug was considered as 10, from the second day, i.e. the day on which administration was started, 10 was considered as the worst, 1 was considered as the mildest, and 0 was consider as normal).

Dosage regimen: 50-100 mg of drug was administered by intravenous injection once a day, and meanwhile, 10 mg of drug was administered by aerosol inhalation twice or three times a day. Administration was performed once every two days for 13 consecutive days.

After 13 days of administration, the patient got 9 points in the memory assessment, 9 points in the computing power assessment, and 9 points in the orientation assessment.

It indicates that plasminogen can improve cognitive impairment, memory function, computing power, orientation ability of the patient.

Patient 5, female, 79-year-old, had symptoms such as poor memory and bad temper 4 years ago, which were gradually worsened. At present, the patient was relatively quiet, with an attention span of no more than 2 minutes, the impaired language expression, and the short-term memory loss. The patient was unable to distinguish time and place and easy to grieve, and had lost self-care ability. The patient got 3 points in MMSE.

Dosage regimen: 50 mg of drug was administered by intravenous injection, the dose was increased by 50 mg every two days, and administration was performed for 14 consecutive days. Then, 400 mg of drug was administered twice a week. Administration was performed for a total of 30 days.

The patient's attention and understanding had improved slightly since the 4th day of administration. On the 7th day of administration, the patient's understanding and attention had further improved, and the patient could understand questions and tried to answer them. On the 10th day of administration, the patient could recognize and remember more things and relatives. On the 14th day of administration, the patient could concentrate for 7-8 minutes; and for what just happened, if the patient was in a good state, the memory could last for 6-7 minutes. On the 21st day of administration, the patient could concentrate for more than 30 minutes. After 24 days of administration, for what just happened, the memory could last for about 1 hour, the language expression was also richer, and the patient got 9 points in MMSE. After 30 days of administration, the patient got 9 points in MMSE. One month after drug withdrawal, the computing power was improved, and the patient got 8 points in MMSE. MMSE scores before and after administration are shown in Table 2.

It shows that plasminogen can increase the MMSE score of the patients with Alzheimer's disease, and improve memory function, cognitive ability, attention, comprehension ability, language competence, and computing power of the patients.

Table 2 MMSE scores before and after administration

|  | MMSE score |
|---|---|
| Before administration | 3 |
| After administration (24 days) | 9 |
| After administration (30 days) | 9 |
| After drug withdrawal (30 days) | 8 |

References

[1] Selkoe D J. Alzheimer's disease: genes, proteins, and therapy [J]. Physiol. Rev, 2001, 81 (2): 741-766.
[2] Oakley H, Cole S L, Logan S, et al. Intraneuronal β-Amyloid Aggregates, Neurodegeneration, and Neuron Loss in Transgenic Mice with Five Familial Alzheimer's Disease Mutations: Potential Factors in Amyloid Plaque Formation [J]. Journal of Neuroscience, 2006, 26 (40): 10129-10140.
[3] Moon M, Choi J G, Kim S Y, et al. Bombycis Excrementum Reduces Amyloid-β Oligomer-Induced Memory Impairments, Neurodegeneration, and Neuroinflammation in Mice [J]. Journal of Alzheimer's disease: JAD, 2014, 41 (2).
[4] Park S W, Kim J H, Mook-Jung I, et al. Intracellular amyloid beta alters he tight junction of retinal pigment epithelium in 5XFAD mice [J]. Neurobiology of Aging, 2014, 35 (9): 2013-2020.
[5] Naseri NN, Wang H, Guo J, Sharma M, Luo W. The complexity of tau in Alzheimer's disease. Neurosci Lett. 2019 Jul 13; 705: 183-194.
[6] Gray K, Ellis V. Activation of pro-BDNF by the pericellular serine protease plasmin [J]. Febs Letters, 2008, 582 (6): 907-910.

[7] Kowiański Przemysiaw, Lietzau G, Czuba E, et al. BDNF: A Key Factor with Multipotent Impact on Brain Signaling and Synaptic Plasticity [J]. Cellular & Molecular Neurobiology, 2017.
[8] Aloe L, Rocco M L, Bianchi P, et al. Nerve growth factor: from the early discoveries to the potential clinical use [J]. Journal of Translational Medicine, 2012, 10 (1).

**Claims**

1. A method for preventing and treating Alzheimer's disease, comprising: administering a therapeutically effective amount of one or more compounds to a subject with Alzheimer's disease, the one or more compounds being selected from: a component of a plasminogen activation pathway, a compound capable of directly activating plasminogen or indirectly activating plasminogen by activating an upstream component of a plasminogen activation pathway, a compound mimicking the activity of plasminogen or plasmin, a compound capable of up-regulating the expression of plasminogen or a plasminogen activator, a plasminogen analog, a plasmin analog, a tPA or uPA analog, and an antagonist of a fibrinolysis inhibitor.

2. The method according to claim 1, wherein the component of the plasminogen activation pathway is selected from plasminogen, recombinant human plasmin, Lys-plasminogen, Glu-plasminogen, plasmin, plasminogen and plasmin variants and analogs containing one or more kringle domains and protease domains of plasminogen and plasmin, mini-plasminogen, mini-plasmin, micro-plasminogen, microplasmin, delta-plasminogen, delta-plasmin, a plasminogen activator, tPA, and uPA.

3. The method according to claim 1, wherein the antagonist of the fibrinolysis inhibitor is an inhibitor of PAI-1, a complement C1 inhibitor, α2 antiplasmin or an α2 macroglobulin, such as an antibody.

4. The method according to any one of claims 1 to 3, wherein the compound has one or more effects on the subject with Alzheimer's disease, and the one or more effects are selected from:
promotion of the degradation of amyloid beta-protein 40 (Aβ40) or amyloid beta-protein 42 (Aβ42) in brain tissue, improvement of memory function, improvement of cognitive ability, improvement of geographical identification ability, relief of anxiety or depression, reduction of Aβ42 deposition in brain tissue, promotion of the degradation of Tau proteins in brain tissue, promotion of the cleavage of Pro-BDNF in brain tissue to form mature BDNF, promotion of the expression of BDNF in brain tissue, promotion of the cleavage of Pro-NGF in brain tissue to form mature NGF, and improvement of hippocampal damage in brain tissue.

5. The method according to any one of claims 1 to 4, wherein the compound is plasminogen.

6. The method according to any one of claims 1 to 5, wherein the plasminogen is human full-length plasminogen or a conservatively substituted variant thereof.

7. The method according to any one of claims 1 to 5, wherein the plasminogen has at least 75%, 80%, 85%, 90%, 95%, 96%, 97%, 98% or 99% sequence identity with sequence 2 and still has the lysine binding activity or the proteolytic activity of plasminogen.

8. The method according to any one of claims 1 to 5, wherein the plasminogen is a protein containing an amino acid sequence that has at least 80%, 90%, 95%, 96%, 97%, 98% or 99% amino acid sequence identity with sequence 14 and still having the proteolytic activity of plasminogen.

9. The method according to any one of claims 1 to 5, wherein the plasminogen is selected from Glu-plasminogen, Lys-plasminogen, mini-plasminogen, micro-plasminogen, delta-plasminogen, and variants thereof that retain the proteolytic activity of plasminogen.

10. The method according to any one of claims 1 to 5, wherein the plasminogen contains an amino acid sequence shown as sequence 2, 6, 8, 10 or 12, or contains a conservatively substituted variant of the amino acid sequences shown as sequence 2, 6, 8, 10 or 12.

11. The method according to any one of claims 1 to 10, wherein the compound is used in combination with one or more other treatment methods or drugs.

12. The method according to claim 11, wherein the other treatment methods comprise a cell therapy (comprising a stem cell therapy), a support therapy, and a physical therapy.

13. The method according to claim 11, wherein the other drugs are other drugs for treating Alzheimer's disease.

14. The method according to any one of claims 1 to 13, wherein the compound is administered by nasal inhalation, aerosol inhalation, nasal drops, eye drops, ear drops, an intravenous method, an intraperitoneal method, a subcutaneous method, an intracranial method, an intrathecal method, an intraarterial method (e.g. via the carotid artery) or an intramuscular method.

Figure 1

Figure 2

Figure 3

Figure 4

Figure 5

Figure 6

Figure 7

Figure 8

Figure 9

Figure 10

Figure 11

Figure 12

Figure 13

Figure 14

Figure 15

Figure 16

Figure 17

Figure 18

Figure 19

Figure 20

Figure 21

Figure 22

Figure 23

Figure 24

Figure 25

Figure 26

Figure 27

Figure 28

## INTERNATIONAL SEARCH REPORT

| International application No. |
| --- |
| **PCT/CN2021/082701** |

| **A.   CLASSIFICATION OF SUBJECT MATTER** |
| --- |
| A61K 38/43(2006.01)i;  A61K 38/49(2006.01)i;  A61P 25/28(2006.01)n |
| According to International Patent Classification (IPC) or to both national classification and IPC |

| **B.   FIELDS SEARCHED** |
| --- |
| Minimum documentation searched (classification system followed by classification symbols) |
| A61K; A61P |
| Documentation searched other than minimum documentation to the extent that such documents are included in the fields searched |
| Electronic data base consulted during the international search (name of data base and, where practicable, search terms used) |
| CNABS, SIPOABS, DWPI, CNKI, Web of Science, Elsevier Science, 超星读秀, SUPERSTAR DUXIU: 纤溶酶原, 纤维蛋白溶酶原, 纤维蛋白溶解酶原, 纤维蛋白溶酶, 阿尔茨海默病, plasminogen, plasmin, Alzheimer disease |

| **C.   DOCUMENTS CONSIDERED TO BE RELEVANT** | | |
| --- | --- | --- |
| Category* | Citation of document, with indication, where appropriate, of the relevant passages | Relevant to claim No. |
| X | WO 0158476 A2 (THE EUROPEAN MOLECULAR BIOLOGY LABORATORY et al.) 16 August 2001 (2001-08-16)<br>see claims 1-12 and 34-36, and description, page 8, paragraph 4, page 17, paragraph 5 and page 21, paragraph 5 | 1-14 |
| X | WO 03071267 A1 (VANDERBILT UNIVERSITY et al.) 28 August 2003 (2003-08-28)<br>see the abstract, and claim 1 | 1, 3, 4 |
| X | US 6471960 B1 (RUTGERS, THE STATE UNIVERSITY) 29 October 2002 (2002-10-29)<br>see claims 1 and 2 | 1, 2, 4 |
| X | US 2014186423 A1 (GELFAND MATHEW) 03 July 2014 (2014-07-03)<br>see claims 1, 2 and 15-17 | 1, 2, 4 |
| X | WO 0162799 A2 (UNIVERSITAIR MEDISCH CENTRUM UTRECHT et al.) 30 August 2001 (2001-08-30)<br>see claims 1, 7 and 8 | 1, 2, 4 |
| X | ELALI Ayman et al. "Tissue-Plasminogen Activator Attenuates Alzheimer's Disease-Related Pathology Development in APPswe/PS1 Mice"<br>*NEUROPSYCHOPHARMACOLOGY*, Vol. 41, No. 5, 09 September 2015 (2015-09-09), pp. 1297-1307, see the abstract | 1, 2, 4 |

| ☑ Further documents are listed in the continuation of Box C. | ☑ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **14 May 2021** | **10 June 2021** |

| Name and mailing address of the ISA/CN | Authorized officer |
| --- | --- |
| **China National Intellectual Property Administration (ISA/ CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

## INTERNATIONAL SEARCH REPORT

International application No.

**PCT/CN2021/082701**

### C.    DOCUMENTS CONSIDERED TO BE RELEVANT

| Category* | Citation of document, with indication, where appropriate, of the relevant passages | Relevant to claim No. |
|---|---|---|
| X | AKHTER Hasina et al. "A Small Molecule Inhibitor of Plasminogen Activator Inhibitor-1 Reduces Brain Amyloid-beta Load and Improves Memory in an Animal Model of Alzheimer's Disease" *JOURNAL OF ALZHEIMERS DISEASE,* Vol. 64, No. 2, 31 December 2018 (2018-12-31), pp. 447-457, see title and the abstract | 1, 3, 4 |
| X | ANGELUCCI Francesco et al. "Amyloid Beta Soluble Forms and Plasminogen Activation System in Alzheimer's Disease: Consequences on Extracellular Maturation of Brain-derived Neurotrophic Factor and Therapeutic Implications" *CNS NEUROSCIENCE & THERAPEUTICS,* Vol. 25, No. 3, 31 March 2019 (2019-03-31), pp. 303-313, see figure 1, sections 9-11 | 1-14 |

Form PCT/ISA/210 (second sheet) (January 2015)

**INTERNATIONAL SEARCH REPORT**

| International application No. |
|---|
| **PCT/CN2021/082701** |

| Box No. I      Nucleotide and/or amino acid sequence(s) (Continuation of item 1.c of the first sheet) |
|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

## INTERNATIONAL SEARCH REPORT

| International application No. |
|---|
| **PCT/CN2021/082701** |

| **Box No. II**      **Observations where certain claims were found unsearchable (Continuation of item 2 of first sheet)** |
|---|

This international search report has not been established in respect of certain claims under Article 17(2)(a) for the following reasons:

1. ☑   Claims Nos.: **1-14**
   because they relate to subject matter not required to be searched by this Authority, namely:

   [1]   The subject matter of claims 1-14 relates to methods of treating human or animal body diseases, and therefore does not warrant an international search according to the criteria set out in PCT Rule 39.1(iv). The subject matter of claim 1 may be reasonably anticipated to be modified to: the application of one or more of the following compounds in the preparation of a drug for the prevention and treatment of Alzheimer's disease, wherein said compound is selected from: a component of the fibrinolysinogen activation pathway, a compound capable of directly activating fibrinolysinogen or indirectly activating fibrinolysinogen by activating an upstream component of the fibrinolysinogen activation pathway, a compound that mimics compounds that mimic the activity of fibrinolysinogen or fibrinolytic enzymes, compounds that upregulate the expression of fibrinolysinogen or fibrinolysinogen activators, fibrinolysinogen analogs, fibrinolytic enzyme analogs, tPA or uPA analogs and antagonists of fibrinolytic inhibitors. The international search is made on the basis of this reasonable anticipation.

2. ☐   Claims Nos.:
   because they relate to parts of the international application that do not comply with the prescribed requirements to such an extent that no meaningful international search can be carried out, specifically:

3. ☐   Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.

**PCT/CN2021/082701**

| Patent document cited in search report | | | Publication date (day/month/year) | Patent family member(s) | | | Publication date (day/month/year) |
|---|---|---|---|---|---|---|---|
| WO | 0158476 | A2 | 16 August 2001 | AU | 3173301 | A | 20 August 2001 |
| | | | | WO | 0158476 | A3 | 28 March 2002 |
| WO | 03071267 | A1 | 28 August 2003 | US | 7057086 | B2 | 06 June 2006 |
| | | | | US | 2003217371 | A1 | 20 November 2003 |
| | | | | EP | 1485709 | A4 | 21 September 2005 |
| | | | | CA | 2476761 | A1 | 28 August 2003 |
| | | | | AU | 2003215315 | A1 | 09 September 2003 |
| | | | | EP | 1485709 | A1 | 15 December 2004 |
| US | 6471960 | B1 | 29 October 2002 | US | 6136548 | A | 24 October 2000 |
| US | 2014186423 | A1 | 03 July 2014 | None | | | |
| WO | 0162799 | A2 | 30 August 2001 | EP | 1257582 | B1 | 29 April 2009 |
| | | | | WO | 0162799 | A3 | 04 April 2002 |
| | | | | AT | 429926 | T | 15 May 2009 |
| | | | | EP | 1257582 | A2 | 20 November 2002 |
| | | | | US | 2006270599 | A1 | 30 November 2006 |
| | | | | DE | 60138524 | D1 | 10 June 2009 |
| | | | | US | 2003050245 | A1 | 13 March 2003 |
| | | | | AU | 4126201 | A | 03 September 2001 |
| | | | | CA | 2400823 | A1 | 30 August 2001 |
| | | | | EP | 1130031 | A1 | 05 September 2001 |

Form PCT/ISA/210 (patent family annex) (January 2015)

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- CN 102154253 A
- WO 9704801 A
- US 3773919 A
- EP 58481 A

## Non-patent literature cited in the description

- **NY, A. ; LEONARDSSON, G. ; HAGGLUND, A.C ; HAGGLOF, P. ; PLOPLIS, V.A. ; CARMELIET, P. ; NY, T.** Ovulation inplasminogen-deficient mice. *Endocrinology,* 1999, vol. 140, 5030-5035
- **SILVERSTEIN RL ; LEUNG LL ; HARPEL PC ; NACHMAN RL.** Complex formation of platelet thrombospondin with plasminogen. Modulation of activation by tissue activator. *J. Clin. Invest.,* November 1984, vol. 74 (5), 1625-33
- **GRAVANIS I ; TSIRKA SE.** Tissue-type plasminogen activator as a therapeutic target in stroke. *Expert Opinion on Therapeutic Targets.,* February 2008, vol. 12 (2), 159-70
- **GEIGER M ; HUBER K ; WOJTA J ; STINGL L ; ESPANA F ; GRIFFIN JH ; BINDER BR.** Complex formation between urokinase and plasma protein C inhibitor in vitro and in vivo. *Blood.,* August 1989, vol. 74 (2), 722-8
- **AISINA R B ; MUKHAMETOVA L I.** Structure and function of plasminogen/plasmin system [J. *Russian Journal of Bioorganic Chemistry,* 2014, vol. 40 (6), 590-605
- **BARANY et al.** Solid-Phase Peptide Synthesis; The Peptides: Analysis, Synthesis, Biology. *Special Methods in Peptide Synthesis, Part A,* vol. 2, 3-284
- **MERRIFIELD.** Solid Phase Peptide Synthesis. I. The Synthesis of a Tetrapeptide. *J. Am. Chem. Soc.,* 1963, vol. 85, 2149-2156
- **STEWART et al.** Solid Phase Peptide Synthesis. Pierce Chem. Co, 1984
- **GANESAN A.** *Mini Rev. Med Chem.,* 2006, vol. 6, 3-10
- **CAMARERO JA et al.** *Protein Pept Lett,* 2005, vol. 12, 723-8
- **WINNACKER.** From Genes to Clones. VCH Publishers, 1987
- **QUEEN et al.** *Immunol. Rev.,* 1986, vol. 89, 49
- **CO et al.** *J. Immunol.,* 1992, vol. 148, 1149
- Remington's Pharmaceutical Sciences. 1980
- **LANGER et al.** *J. Biomed. Mater. Res.,* 1981, vol. 15, 167-277
- **LANGER.** *Chem. Tech.,* 1982, vol. 12, 98-105
- **SIDMAN et al.** *Biopolymers,* 1983, vol. 22, 547
- **KENNETH C ROBBINS ; LOUIS SUMMARIA ; DAVID ELWYN et al.** Further Studies on the Purification and Characterization of Human Plasminogen and Plasmin. *Journal of Biological Chemistry,* 1965, vol. 240 (1), 541-550
- **SUMMARIA L ; SPITZ F ; ARZADON L et al.** Isolation and characterization of the affinity chromatography forms of human Glu- and Lys-plasminogens and plasmins. *J Biol Chem.,* 25 June 1976, vol. 251 (12), 3693-9
- **HAGAN JJ ; ABLONDI FB ; DE RENZO EC.** Purification and biochemical properties of human plasminogen. *J Biol Chem.,* April 1960, vol. 235, 1005-10
- **SELKOE D J.** Alzheimer's disease: genes, proteins, and therapy [J. *Physiol. Rev,* 2001, vol. 81 (2), 741-766
- **OAKLEY H ; COLE S L ; LOGAN S et al.** Intraneuronal β-Amyloid Aggregates, Neurodegeneration, and Neuron Loss in Transgenic Mice with Five Familial Alzheimer's Disease Mutations: Potential Factors in Amyloid Plaque Formation [J. *Journal of Neuroscience,* 2006, vol. 26 (40), 10129-10140
- **MOON M ; CHOI J G ; KIM S Y et al.** Bombycis Excrementum Reduces Amyloid-β Oligomer-Induced Memory Impairments, Neurodegeneration, and Neuroinflammation in Mice [J. *Journal of Alzheimer's disease: JAD,* 2014, vol. 41 (2
- **PARK S W ; KIM J H ; MOOK-JUNG I et al.** Intracellular amyloid beta alters he tight junction of retinal pigment epithelium in 5XFAD mice [J. *Neurobiology of Aging,* 2014, vol. 35 (9), 2013-2020
- **NASERI NN ; WANG H ; GUO J ; SHARMA M ; LUO W.** The complexity of tau in Alzheimer's disease. *Neurosci Lett.,* 13 July 2019, vol. 705, 183-194
- **GRAY K ; ELLIS V.** Activation of pro-BDNF by the pericellular serine protease plasmin [J. *Febs Letters,* 2008, vol. 582 (6), 907-910

- **LIETZAU G ; CZUBA E et al.** BDNF: A Key Factor with Multipotent Impact on Brain Signaling and Synaptic Plasticity [J. *Cellular & Molecular Neurobiology,* 2017

- **ALOE L ; ROCCO M L ; BIANCHI P et al.** Nerve growth factor: from the early discoveries to the potential clinical use [J. *Journal of Translational Medicine,* 2012, vol. 10 (1